# EUROPEAN PATENT SPECIFICATION

(11) **EP 4 010 343 B1**
(45) Date of publication and mention of the grant of the patent: **13.09.2023**
(21) Application number: 20757193.6
(22) Date of filing: 03.08.2020
(51) Int. Cl.: C07D 487/04, A61P 35/00, A61K 31/55

(54) **7,8-DIHYDRO-4H-PYRAZOLO[4,3-C]AZEPINE-6-ONE COMPOUNDS**
7,8-DIHYDRO-4H-PYRAZOLO[4,3-C] AZEPIN-6-ON-VERBINDUNGEN
COMPOSÉS DE 7,8-DIHYDRO-4H-PYRAZOLO [4,3-C] AZÉPINE-6-ONE

(30) Priority: 05.08.2019 EP 19382680
(43) Date of publication of application: 15.06.2022
(73) Proprietor: Eli Lilly and Company, Indianapolis, IN 46285 (US)
(72) Inventor: LUMERAS AMADOR, Wenceslao, Indianapolis, Indiana 46206-6288 (US); BRANDHUBER, Barbara Jean, Indianapolis, Indiana 46206-6288 (US); IYER, Chandrasekar Venkataraman, Indianapolis, (US); LALLENA JIMENO, Maria Jose, Madrid (ES); SHAHDA, Safi, Indianapolis (US)
(74) Representative: Ingham, Stephen Howard
(86) International application number: PCT/US2020/044718
(87) International publication number: WO 2021/026059

(56) References cited:
- WO-A1-2017/205538
- US-A1- 2017 333 406

## Description

The present invention relates to certain novel compounds that are CREBBP and EP300 bromodomain dual inhibitors, to pharmaceutical compositions comprising the compounds, to methods of using the compounds to treat certain cancers, and processes useful in the synthesis of the compounds.

The present invention is in the field of treating cancer. CREBBP and EP300 are histone acetyltransferases (HAT) and function as coactivators for a number of transcription factors. CREBBP and EP300 bromodomain dual inhibitors have been reported to mediate antiproliferative responses in hematologic cancer cell lines, such as acute myeloid leukemia (AML) (See, e.g., T.D. Crawford, et al., J. Med.Chem., vol. 59, pages 10549-10563 (2016)), and multiple myeloma cell lines (See, e.g., A.R. Conery, eLIFE 2016;5:e10483). There continues to be an unmet need in the treatment of cancer, and new treatment options are desired.

WO 2017/205538 A1 discloses certain pyrazolopyridine derivatives that are inhibitors of CBP and/or EP300, and are useful in the treatment of various CBP and/or EP300 mediated disorders such as cancer, inflammatory disorders, and autoimmune diseases. US 2017/0333406 A1 discloses certain pyrazolopyridine derivatives that are inhibitors of CBP and/or EP300 which are useful in the treatment of CBP and /or EP300 mediated disorders.

Additional CREBBP and EP300 bromodomain dual inhibitor compounds are desired. The present invention provides certain compounds that inhibit at least one of CREBBP and EP300 bromodomain and preferably, are dual inhibitors of the CREBBP and EP300 bromodomain. In addition, the present invention provides compounds that are selective dual inhibitors of the CREBBP and EP300 bromodomain over the bromodomain-containing protein 4 ("BRD4").

The present invention further provides compounds that are useful for treating cancers, such as squamous cell cancers, bladder urothelial cancer, breast cancer, colon cancer, diffuse large B cell lymphoma (DLBCL), follicular lymphoma, marginal zone lymphoma, upper tract urothelial cancer, endometrial carcinoma, uterine endometrioid carcinoma, SCLC, prostate cancer, and lymphoma.

In addition, the compounds of the present invention may be useful for treating tumors with mutations of CREBBP and/or EP300 such as esophageal cancer, lung cancer, SCLC, lymphoma, bladder cancer, squamous histology, squamous cell cancers including anal, cervical, head and neck, lung, and oropharynx cancers, uterine endometrioid, and HPV driven cancers. The compounds disclosed herein may also be useful for treating AML-ETO translocation related conditions, androgen receptor positive related conditions, myc overexpression related conditions, acute myeloid leukemia (including AML-ETO fusions), acute lymphoblastic leukemia, multiple myelomas, and prostate cancer. Squamous cell cancers include anal cancer, bladder cancer, cervical cancer, head and neck cancer, lung cancer, and oropharynx cancer. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Accordingly, the present invention provides a compound of Formula I: 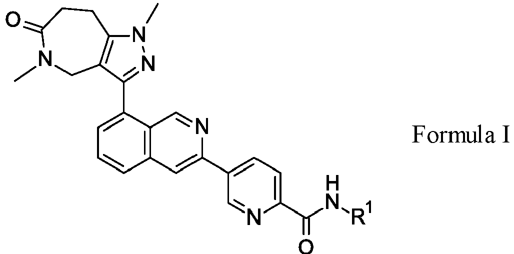
wherein R¹ is -CH₃ or -CH₂CH₃;
or a pharmaceutically acceptable salt thereof.

The present invention also provides a method of treating cancer in a patient, comprising administering to a patient in need of such treatment an effective amount of a compound of Formula I, or a pharmaceutically acceptable salt thereof. Cancers that can be treated include, but are not limited to squamous cell cancer, bladder urothelial cancer, breast cancer, colon cancer, diffuse large B cell lymphoma (DLBCL), follicular lymphoma, marginal zone lymphoma, upper tract urothelial cancer, endometrial carcinoma, uterine endometrioid carcinoma, SCLC, prostate cancer, lymphoma, esophageal cancer, lung cancer, bladder cancer, squamous histology, uterine endometrioid, HPV driven cancers, AML-ETO translocation related conditions, androgen receptor positive related conditions, myc overexpression related conditions, acute myeloid leukemia, acute lymphoblastic leukemia, multiple myelomas, human papillomavirus driven cancer, and prostate cancer.

The present invention provides a method of treating small cell lung cancer (SCLC) in a patient, comprising administering to a patient in need of such treatment an effective amount of a compound of Formula I, or a pharmaceutically acceptable salt thereof. The present invention also provides a method of treating lymphoma in a patient, comprising administering to a patient in need of such treatment an effective amount of a compound of Formula I, or a pharmaceutically acceptable salt thereof. The present invention also provides a method of treating squamous cell cancers in a patient, comprising administering to a patient in need of such treatment an effective amount of a compound of Formula I, or a pharmaceutically acceptable salt thereof (See Vettore, AL., Ramnarayanan, K., Poore, G. et al. Mutational landscapes of tongue carcinoma reveal recurrent mutations in genes of therapeutic and prognostic relevance. Genome Med. 7 (1) 98 (2015) (notes CREBBP gene alterations in oral tongue squamous cell carcinoma); See also Martin D, Abba MC, Molinolo AA, et al. The head and neck cancer cell oncogenome: a platform for the development of precision molecular therapies. Oncotarget. 5 (19):8906-8923. (2014) (notes CREBBP and EP300 gene alterations in head and neck squamous cell carcinoma)). The present invention also provides a method of treating human papillomavirus driven cancers in a patient, comprising administering to a patient in need of such treatment an effective amount of a compound of Formula I, or a pharmaceutically acceptable salt thereof.

Also disclosed is a method of treating tumors with mutations of CREBBP and/or EP300 in a patient, comprising administering to a patient in need of such treatment an effective amount of a compound of Formula I, or a pharmaceutically acceptable salt thereof. Disclosed is a method of treating bladder urothelial cancer in a patient, comprising administering to a patient in need of such treatment an effective amount of a compound of Formula I, or a pharmaceutically acceptable salt thereof (See Gui, Y., Guo, G., Huang, Y. et al. Frequent mutations of chromatin remodeling genes in transitional cell carcinoma of the bladder. Nature Genetics 43 (9), 875-8 (2011) (notes CREBBP andEP300 genetic aberrations in bladder cancer patients)). Further, disclosed is a method of treating breast cancer in a patient, comprising administering to a patient in need of such treatment an effective amount of a compound of Formula I, or a pharmaceutically acceptable salt thereof. Disclosed is a method of treating colon cancer in a patient, comprising administering to a patient in need of such treatment an effective amount of a compound of Formula I, or a pharmaceutically acceptable salt thereof (See Bryan, EJ., Jokubaitis, VJ., Chamberlain, NL., et al. Mutation analysis of EP300 in colon, breast and ovarian carcinomas. Int J Cancer. 102(2): 137-141 (2002) (notes EP300 genetic mutations in human colon, breast, and ovarian cancers)). Furthermore, disclosed is a method of treating diffuse large B cell lymphoma (DLBCL) in a patient, comprising administering to a patient in need of such treatment an effective amount of a compound of Formula I, or a pharmaceutically acceptable salt thereof (See Morin, RD., Mendez-Lago, M., Mungall, AJ. et al. Frequent mutation of histone-modifying genes in non-Hodgkin lymphoma. Nature. 476 (7360) 298-303 (notes CREBBP and EP300 genetic mutations in non-Hodgkin lymphoma). Disclosed is a method of treating follicular lymphoma in a patient, comprising administering to a patient in need of such treatment an effective amount of a compound of Formula I, or a pharmaceutically acceptable salt thereof (See Okosun J, Bödör C, Wang J, et al. Integrated genomic analysis identifies recurrent mutations and evolution patterns driving the initiation and progression of follicular lymphoma. Nature Genetics 46 (2) 176-181 (2014) (notes CREBBP genetic mutations in follicular lymphoma)). Disclosed is a method of treating marginal zone lymphoma in a patient, comprising administering to a patient in need of such treatment an effective amount of a compound of Formula I, or a pharmaceutically acceptable salt thereof (See Jung, H., Yoo, HY., Lee, SH, et al. The mutational landscape of ocular marginal zone lymphoma identifies frequent alterations in TNFAIP3 followed by mutations in TBL1XR1 and CREBBP. Oncotarget 8 (10) 17038-17049 (2017) (notes CREBBP genetic mutations in ocular marginal zone lymphoma)). Disclosed is a method of treating upper tract urothelial cancer, in a patient, comprising administering to a patient in need of such treatment an effective amount of a compound of Formula I, or a pharmaceutically acceptable salt thereof (See Grivas, P., Mortazavi, A., Picus, J. et al. Mocetinostat for patients with previously treated, locally advanced/metastatic urothelial carcinoma and inactivating alterations of acetyltransferase genes. Cancer. 125 (4) 533-540. (2019) (notes CREBBP and EP300 genetic mutations in advanced/metastatic urothelial carcinoma)). Disclosed is a method of treating endometrial carcinoma in a patient, comprising administering to a patient in need of such treatment an effective amount of a compound of Formula I, or a pharmaceutically acceptable salt thereof. Disclosed is a method of treating uterine endometrioid carcinoma in a patient, comprising administering to a patient in need of such treatment an effective amount of a compound of Formula I, or a pharmaceutically acceptable salt thereof. Disclosed is a method of treating SCLC in a patient, comprising administering to a patient in need of such treatment an effective amount of a compound of Formula I, or a pharmaceutically acceptable salt thereof. Disclosed is a method of treating prostate cancer in a patient, comprising administering to a patient in need of such treatment an effective amount of a compound of Formula I, or a pharmaceutically acceptable salt thereof (See Liu, J., He, D., Cheng, L. et al. p300/CBP inhibition enhances the efficacy of programmed death-ligand 1 blockade treatment in prostate cancer. Oncogene 39, 3939-3951 (2020) (notes the effect of CREBBP and EP300 inhibitors in treating prostate cancer)).

Also disclosed is a method of treating tumors with mutations of CREBBP and/or EP300 such as esophageal cancer, lung cancer, SCLC, lymphoma, bladder cancer, and squamous histology in a patient, comprising administering to a patient in need of such treatment an effective amount of a compound of Formula I, or a pharmaceutically acceptable salt thereof. Disclosed is a method of treating squamous cell cancers, in a patient, comprising administering to a patient in need of such treatment an effective amount of a compound of Formula I, or a pharmaceutically acceptable salt thereof (See Morris, V., Rao, X., Pickering, C. et al. Comprehensive Genomic Profiling of Metastatic Squamous Cell Carcinoma of the Anal Canal. Molecular Cancer Research 15 (11), 1542-1550 (2017) (notes EP300 genetic mutations in Squamous cell carcinoma of the anal canal); Vettore, AL., Ramnarayanan, K., Poore, G. et al. Mutational landscapes of tongue carcinoma reveal recurrent mutations in genes of therapeutic and prognostic relevance. Genome Med. 7 (1) 98 (2015) (notes CREBBP and EP300 genetic mutations in carcinoma of the oral tongue); and Martin D, Abba MC, Molinolo AA, et al. The head and neck cancer cell oncogenome: a platform for the development of precision molecular therapies. Oncotarget. 5 (19):8906-8923. (2014) (notes CREBBP and EP300 genetic mutations in head and neck squamous cell carcinoma)). Further disclosed is a method of treating uterine endometrioid in a patient, comprising administering to a patient in need of such treatment an effective amount of a compound of Formula I, or a pharmaceutically acceptable salt thereof. Disclosed is a method of treating HPV driven cancers in a patient, comprising administering to a patient in need of such treatment an effective amount of a compound of Formula I, or a pharmaceutically acceptable salt thereof. Disclosed is a method of treating AML-ETO translocation related conditions in a patient, comprising administering to a patient in need of such treatment an effective amount of a compound of Formula I, or a pharmaceutically acceptable salt thereof (See Zhang, J., Kalkum, M., Yamamura, S. et al. E protein silencing by the leukemogenic AML1-ETO fusion protein. Science. 305 (5688) 1286-1289 (2004) (notes CREBBP and EP300 genetic mutations in acute myeloid leukemia cases)). Disclosed is a method of treating androgen receptor positive related conditions in a patient, comprising administering to a patient in need of such treatment an effective amount of a compound of Formula I, or a pharmaceutically acceptable salt thereof (See Garcia-Carpizo, V., Ruiz-Llorente, S., Sarmentero, J. et al. CREBBP/EP300 Bromodomain Inhibition Affects the Proliferation of AR-Positive Breast Cancer Cell Lines. Molecular Cancer Research 17 (3), 720-730 (2019) (notes the effect of CREBBP and EP300 inhibitors in androgen receptor positive breast cancer cell lines); See also Aarnisalo, P., Palvimo, JJ. & Jänne, OA. et al. CREB-binding protein in androgen receptor-mediated signaling. PNAS 95 (5), 2122-2127 (1998) (notes the presence of CREBBP in androgen-dependent signaling pathways)). Disclosed is a method of treating myc overexpression related conditions in a patient, comprising administering to a patient in need of such treatment an effective amount of a compound of Formula I, or a pharmaceutically acceptable salt thereof.

Disclosed is a method of treating acute myeloid leukemia, including AML-ETO fusions in a patient, comprising administering to a patient in need of such treatment an effective amount of a compound of Formula I, or a pharmaceutically acceptable salt thereof (See Brooks et al, CCS1477: A Novel Small Molecule Inhibitor of p300/CBP Bromodomain for the Treatment of Acute Myeloid Leukaemia and Multiple Myeloma. Blood (2019) 134 (Supplement 1): 2560) (notes the effect of CREBBP and EP300 inhibitors in AML treatment)). Disclosed is a method of treating acute lymphoblastic leukemia in a patient, comprising administering to a patient in need of such treatment an effective amount of a compound of Formula I, or a pharmaceutically acceptable salt thereof (See Qian, M., Zhang, H., Kham, SK. et al. Whole-transcriptome sequencing identifies a distinct subtype of acute lymphoblastic leukemia with predominant genomic abnormalities of EP300 and CREBBP. Genome Research (2017) 27 185-195) (notes CREBBP and EP300 genetic mutations in acute lymphoblastic leukemia cases). Also disclosed is a method of treating multiple myelomas in a patient, comprising administering to a patient in need of such treatment an effective amount of a compound of Formula I, or a pharmaceutically acceptable salt thereof (See Brooks et al, CCS1477: A Novel Small Molecule Inhibitor of p300/CBP Bromodomain for the Treatment of Acute Myeloid Leukaemia and Multiple Myeloma. Blood (2019) 134 (Supplement 1): 2560). Disclosed is a method of treating prostate cancer in a patient, comprising administering to a patient in need of such treatment an effective amount of a compound of Formula I, or a pharmaceutically acceptable salt thereof (See Liu, J., He, D., Cheng, L. et al. p300/CBP inhibition enhances the efficacy of programmed death-ligand 1 blockade treatment in prostate cancer. Oncogene 39, 3939-3951 (2020) (notes the effect of CREBBP and EP300 inhibitors in prostate cancer treatment)).

Furthermore, this invention provides a compound of Formula I, or a pharmaceutically acceptable salt thereof for use in therapy. This invention also provides a compound of Formula I, or a pharmaceutically acceptable salt thereof, for use in treating cancer. Cancers that can be treated include squamous cell cancer, bladder urothelial cancer, breast cancer, colon cancer, diffuse large B cell lymphoma (DLBCL), follicular lymphoma, marginal zone lymphoma, upper tract urothelial cancer, endometrial carcinoma, uterine endometrioid carcinoma, SCLC, prostate cancer, lymphoma, esophageal cancer, lung cancer, bladder cancer, squamous histology, uterine endometrioid, HPV driven cancers, AML-ETO translocation related conditions, androgen receptor positive related conditions, myc overexpression related conditions, acute myeloid leukemia, acute lymphoblastic leukemia, multiple myelomas, human papillomavirus driven cancer, and prostate cancer.

This invention also provides a compound of Formula I, or a pharmaceutically acceptable salt thereof, for use in treating small cell lung cancer. In addition, this invention provides a compound of Formula I, or a pharmaceutically acceptable salt thereof, for use in treating lymphoma. In addition, this invention provides a compound of Formula I, or a pharmaceutically acceptable salt thereof, for use in treating squamous cell cancer. In addition, this invention provides a compound of Formula I, or a pharmaceutically acceptable salt thereof, for use in treating human papillomavirus driven cancers.

This invention also provides a compound of Formula I, or a pharmaceutically acceptable salt thereof, for use in treating squamous cell cancers. This invention also provides a compound of Formula I, or a pharmaceutically acceptable salt thereof, for use in treating bladder urothelial cancer. This invention also provides a compound of Formula I, or a pharmaceutically acceptable salt thereof, for use in treating breast cancer. This invention also provides a compound of Formula I, or a pharmaceutically acceptable salt thereof, for use in treating colon cancer. This invention also provides a compound of Formula I, or a pharmaceutically acceptable salt thereof, for use in treating diffuse large B cell lymphoma (DLBCL). This invention also provides a compound of Formula I, or a pharmaceutically acceptable salt thereof, for use in treating follicular lymphoma. This invention also provides a compound of Formula I, or a pharmaceutically acceptable salt thereof, for use in treating marginal zone lymphoma. This invention also provides a compound of Formula I, or a pharmaceutically acceptable salt thereof, for use in treating upper tract urothelial cancer. This invention also provides a compound of Formula I, or a pharmaceutically acceptable salt thereof, for use in treating endometrial carcinoma. This invention also provides a compound of Formula I, or a pharmaceutically acceptable salt thereof, for use in treating uterine endometrioid carcinoma. This invention also provides a compound of Formula I, or a pharmaceutically acceptable salt thereof, for use in treating SCLC. This invention also provides a compound of Formula I, or a pharmaceutically acceptable salt thereof, for use in treating prostate cancer. And this invention also provides a compound of Formula I, or a pharmaceutically acceptable salt thereof, for use in treating lymphoma.

In addition, this invention also provides a compound of Formula I, or a pharmaceutically acceptable salt thereof, for use in treating tumors with mutations of CREBBP and/or EP300. This invention also provides a compound of Formula I, or a pharmaceutically acceptable salt thereof, for use in treating esophageal cancer. This invention also provides a compound of Formula I, or a pharmaceutically acceptable salt thereof, for use in treating lung cancer. This invention also provides a compound of Formula I, or a pharmaceutically acceptable salt thereof, for use in treating SCLC. This invention also provides a compound of Formula I, or a pharmaceutically acceptable salt thereof, for use in treating lymphoma. This invention also provides a compound of Formula I, or a pharmaceutically acceptable salt thereof, for use in treating bladder cancer. This invention also provides a compound of Formula I, or a pharmaceutically acceptable salt thereof, for use in treating squamous histology. This invention also provides a compound of Formula I, or a pharmaceutically acceptable salt thereof, for use in treating, squamous cell cancers. This invention also provides a compound of Formula I, or a pharmaceutically acceptable salt thereof, for use in treating uterine endometrioid driven cancers. This invention also provides a compound of Formula I, or a pharmaceutically acceptable salt thereof, for use in treating HPV driven cancers. This invention also provides a compound of Formula I, or a pharmaceutically acceptable salt thereof, for use in treating AML-ETO translocation related conditions. This invention also provides a compound of Formula I, or a pharmaceutically acceptable salt thereof, for use in treating androgen receptor positive related conditions. This invention also provides a compound of Formula I, or a pharmaceutically acceptable salt thereof, for use in treating myc overexpression related conditions. This invention also provides a compound of Formula I, or a pharmaceutically acceptable salt thereof, for use in treating acute myeloid leukemia (including AML-ETO fusions). This invention also provides a compound of Formula I, or a pharmaceutically acceptable salt thereof, for use in treating acute lymphoblastic leukemia. This invention also provides a compound of Formula I, or a pharmaceutically acceptable salt thereof, for use in treating multiple myelomas. This invention also provides a compound of Formula I, or a pharmaceutically acceptable salt thereof, for use in treating prostate cancer.

This invention provides the use of a compound of Formula I, or a pharmaceutically acceptable salt thereof, for the manufacture of a medicament for treating cancer. Cancers that can be treated include squamous cell cancer, bladder urothelial cancer, breast cancer, colon cancer, diffuse large B cell lymphoma (DLBCL), follicular lymphoma, marginal zone lymphoma, upper tract urothelial cancer, endometrial carcinoma, uterine endometrioid carcinoma, SCLC, prostate cancer, lymphoma, esophageal cancer, lung cancer, bladder cancer, squamous histology, uterine endometrioid, HPV driven cancers, AML-ETO translocation related conditions, androgen receptor positive related conditions, myc overexpression related conditions, acute myeloid leukemia, acute lymphoblastic leukemia, multiple myelomas, human papillomavirus driven cancer, and prostate cancer.

In addition, this invention provides the use of a compound of Formula I, or a pharmaceutically acceptable salt thereof, for the manufacture of a medicament for treating small cell lung cancer (SCLC). This invention also provides the use of a compound of Formula I, or a pharmaceutically acceptable salt thereof, for the manufacture of a medicament for treating lymphoma. This invention also provides the use of a compound of Formula I, or a pharmaceutically acceptable salt thereof, for the manufacture of a medicament for treating squamous cell cancer. This invention also provides the use of a compound of Formula I, or a pharmaceutically acceptable salt thereof, for the manufacture of a medicament for treating human papillomavirus driven cancers.

This invention also provides the use of a compound of Formula I, or a pharmaceutically acceptable salt thereof, for the manufacture of a medicament for treating squamous cell cancers. This invention also provides the use of a compound of Formula I, or a pharmaceutically acceptable salt thereof, for the manufacture of a medicament for treating bladder urothelial cancer. This invention also provides the use of a compound of Formula I, or a pharmaceutically acceptable salt thereof, for the manufacture of a medicament for treating breast cancer. This invention also provides the use of a compound of Formula I, or a pharmaceutically acceptable salt thereof, for the manufacture of a medicament for treating colon cancer. This invention also provides the use of a compound of Formula I, or a pharmaceutically acceptable salt thereof, for the manufacture of a medicament for treating diffuse large B cell lymphoma (DLBCL). This invention also provides the use of a compound of Formula I, or a pharmaceutically acceptable salt thereof, for the manufacture of a medicament for treating follicular lymphoma. This invention also provides the use of a compound of Formula I, or a pharmaceutically acceptable salt thereof, for the manufacture of a medicament for treating marginal zone lymphoma. This invention also provides the use of a compound of Formula I, or a pharmaceutically acceptable salt thereof, for the manufacture of a medicament for treating upper tract urothelial cancer. This invention also provides the use of a compound of Formula I, or a pharmaceutically acceptable salt thereof, for the manufacture of a medicament for treating endometrial carcinoma. This invention also provides the use of a compound of Formula I, or a pharmaceutically acceptable salt thereof, for the manufacture of a medicament for treating uterine endometrioid carcinoma. This invention also provides the use of a compound of Formula I, or a pharmaceutically acceptable salt thereof, for the manufacture of a medicament for treating SCLC. This invention also provides the use of a compound of Formula I, or a pharmaceutically acceptable salt thereof, for the manufacture of a medicament for treating prostate cancer. This invention also provides the use of a compound of Formula I, or a pharmaceutically acceptable salt thereof, for the manufacture of a medicament for treating lymphoma.

This invention also provides the use of a compound of Formula I, or a pharmaceutically acceptable salt thereof, for the manufacture of a medicament for treating tumors with mutations of CREBBP and/or EP300. This invention also provides the use of a compound of Formula I, or a pharmaceutically acceptable salt thereof, for the manufacture of a medicament for treating esophageal cancer. This invention also provides the use of a compound of Formula I, or a pharmaceutically acceptable salt thereof, for the manufacture of a medicament for treating lung cancer. This invention also provides the use of a compound of Formula I, or a pharmaceutically acceptable salt thereof, for the manufacture of a medicament for treating SCLC. This invention also provides the use of a compound of Formula I, or a pharmaceutically acceptable salt thereof, for the manufacture of a medicament for treating lymphoma. This invention also provides the use of a compound of Formula I, or a pharmaceutically acceptable salt thereof, for the manufacture of a medicament for treating bladder cancer. This invention also provides the use of a compound of Formula I, or a pharmaceutically acceptable salt thereof, for the manufacture of a medicament for treating squamous histology. This invention also provides the use of a compound of Formula I, or a pharmaceutically acceptable salt thereof, for the manufacture of a medicament for treating squamous cell cancers including anal, cervical, head and neck, lung, and oropharynx cancers. This invention also provides the use of a compound of Formula I, or a pharmaceutically acceptable salt thereof, for the manufacture of a medicament for treating uterine endometrioid driven cancers. This invention also provides the use of a compound of Formula I, or a pharmaceutically acceptable salt thereof, for the manufacture of a medicament for treating HPV driven cancers. This invention also provides the use of a compound of Formula I, or a pharmaceutically acceptable salt thereof, for the manufacture of a medicament for treating AML-ETO translocation related conditions. This invention also provides the use of a compound of Formula I, or a pharmaceutically acceptable salt thereof, for the manufacture of a medicament for treating androgen receptor positive related conditions. This invention also provides the use of a compound of Formula I, or a pharmaceutically acceptable salt thereof, for the manufacture of a medicament for treating myc overexpression related conditions. This invention also provides the use of a compound of Formula I, or a pharmaceutically acceptable salt thereof, for the manufacture of a medicament for treating acute myeloid leukemia (including AML-ETO fusions). This invention also provides the use of a compound of Formula I, or a pharmaceutically acceptable salt thereof, for the manufacture of a medicament for treating acute lymphoblastic leukemia. This invention also provides the use of a compound of Formula I, or a pharmaceutically acceptable salt thereof, for the manufacture of a medicament for treating multiple myelomas. This invention also provides the use of a compound of Formula I, or a pharmaceutically acceptable salt thereof, for the manufacture of a medicament for treating prostate cancer.

The invention further provides a pharmaceutical composition, comprising a compound of Formula I, or a pharmaceutically acceptable salt thereof, with one or more pharmaceutically acceptable carriers, diluents, or excipients. The invention further provides a process for preparing a pharmaceutical composition, comprising admixing a compound of Formula I, or a pharmaceutically acceptable salt thereof, with one or more pharmaceutically acceptable carriers, diluents, or excipients. This invention also encompasses novel intermediates and processes for the synthesis of the compounds of Formula I.

As used herein, the terms "treating", "treatment", or "to treat" includes restraining, slowing, stopping, or reversing the progression or severity of an existing symptom or disorder.

As used herein, the term "patient" refers to a mammal, in particular a human.

As used herein, the term "effective amount" refers to the amount or dose of compound of the invention, or a pharmaceutically acceptable salt thereof which, upon single or multiple dose administration to the patient, provides the desired effect in the patient under diagnosis or treatment.

An effective amount can be determined by one skilled in the art by the use of known techniques and by observing results obtained under analogous circumstances. In determining the effective amount for a patient, a number of factors are considered by the attending diagnostician, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

The compounds of the present invention are formulated as pharmaceutical compositions administered by any route which makes the compound bioavailable. Most preferably, such compositions are for oral administration. Such pharmaceutical compositions and processes for preparing same are well known in the art (See, e.g., Remington: The Science and Practice of Pharmacy, L.V. Allen, Editor, 22nd Edition, Pharmaceutical Press, 2012).

Certain intermediates described in the following preparations may contain one or more nitrogen protecting groups. As used herein, "PG" refers to a suitable protecting group. It is understood that protecting groups may be varied as appreciated by one of skill in the art depending on the particular reaction conditions and the particular transformations to be performed. The protection and deprotection conditions are well known to the skilled artisan and are described in the literature (See for example *"*Greene's Protective Groups in Organic Synthesis", Fourth Edition, by Peter G.M. Wuts and Theodora W. Greene, John Wiley and Sons, Inc. 2007).

In one aspect, the compounds and/or salts described herein are crystalline. In an embodiment, the compounds disclosed herein are free amines. In a further asepct, the free amine is crystalline. Alternatively, the free amine may be converted into a pharmacuetically acceptable salt, as disclosed below.

In one aspect, the compounds and/or salts described herein are administered in combination with one or more other medicaments. The compounds and/or salts described herein and the one or more other medicaments may be administered at the same time or within an hour or so of each other. Non-limiting examples of other medicaments for combination with the compounds and/or salts described herein include other anti-cancer drugs, steroids, and anti-inflammatories. Non-limiting examples of other anti-cancer drugs include darolutamide, enzalutamide, abemaciclib, alpelisib, and BRD4 inhibitors. Non-limiting examples of BRD4 inhibitors include CPI-0610, PLX51107, GSK2820151(I-BET151), OTX015(MK-8628), ABBV-075, FT-1101, GSK525762(I-BET762), ZEN003694, and ABBV-744. In an embodiment, the compounds and/or salts described herein and the one or more other medicaments are formulated as a single dosage form. In other embodiment, they are formulated separately.

A pharmaceutically acceptable salt of a compound of the invention can be formed, for example, by reaction of an appropriate free base of a compound of the invention, an appropriate pharmaceutically acceptable acid in a suitable solvent such as diethyl ether under standard conditions well known in the art. Additionally, the formation of such pharmaceutically acceptable salts can occur simultaneously upon deprotection of a nitrogen protecting group. See, for example, Gould, P.L., "Salt selection for basic drugs," International Journal of Pharmaceutics, 33: 201-217 (1986); Bastin, R.J., et al. "Salt Selection and Optimization Procedures for Pharmaceutical New Chemical Entities," Organic Process Research and Development, 4: 427-435 (2000); and Berge, S.M., et al., "Pharmaceutical Salts," Journal of Pharmaceutical Sciences, 66: 1-19, (1977).

Certain abbreviations are defined as follows: "ACN" refers to acetonitrile; "AR" refers to androgen receptor; "aq" refers to aqueous; "bis(pinacolato)diboron" refers to bis(pinacolato)diborane or 4,4,5,5-tetramethyl-2-(4,4,5,5-tetramethyl-1,3,2-dioxaborolan-2-yl)-1,3,2-dioxaborolane; "cAMP" refers to cyclic adenosine monophosphate; "cataCXium^{®} A Pd G3" refers to mesylate[(di(1-adamantyl)-n-butylphosphine)-2-(2'-amino-1,1'-biphenyl)]palladium(II) or [(di(1-adamantyl)-butylphosphine)-2-(2'-amino-1,1'-biphenyl)]palladium(II) methanesulfonate; "CBP", "CREB-binding protein", and "CREBBP" can be used interchangeably and refer to cAMP response element binding protein-binding protein; "CDI" refers 1,1'-carbonyldiimidazole; "CPME" refers to cyclopentyl methyl ether; "DCC" refers to 1,3-dicyclohexylcarbodiimide; "DCM" refers to dichloromethane or methylene chloride; "DIC" refers to 1,3-diisopropylcarbodiimide; "DIPEA" refers to N,N-diisopropylethylamine or N-ethyl-N-isopropyl-propan-2-amine; "DMAP" refers to 4-dimethylaminopyridine; "DMF" refers to N,N-dimethylformamide; "DMEM" refers to Dulbecco's Modified Eagle's Medium; "DMSO" refers to dimethyl sulfoxide; "DNA" refers to deoxyribonucleic acid; "EDCI" refers to 1-(3-dimethylaminopropyl)-3-ethylcarbodiimide hydrochloride; "EtOAc" refers to ethyl acetate; "EtOH" refers to ethyl alcohol or ethanol; "FBS" refers to Fetal Bovine Serum; "HATU" refers to 1-[bis(dimethylamino)methylene]-1H-1,2,3-triazolo[4,5-b]pyridinium 3-oxide hexafluorophosphate; "HBTU" refers to 3-[bis(dimethylamino)methyliumyl]-3H-benzotriazol-1-oxide hexafluorophosphate; "HEK" refers to human embryonic kidney; "HOAt" refers to 1-hydroxy-7-azobenzotriazole; "HOBt" refers to 1-hydroxylbenzotriazole hydrate; "HPLC" refers to high performance liquid chromatography; "iPrOH" refers to isopropanol or isopropyl alcohol; "KOAc" refers to potassium acetate; "MEM" refers to Minimum Essential Medium; "MeOH" refers to methanol or methyl alcohol; "MTBE" refers to methyl t-butyl ether; "ONf ' refers to nonaflate or CF₃(CF₂)₃SO₃-; "PBS" refers to Phosphate Buffered Saline; "Pd₂(dba)₃" refers to tris(dibenzylideneacetone)dipalladium(0); "Pd(dppf)Cl₂" refers to [1,1'-bis(diphenylphosphino)ferrocene]dichloropalladium(II); "Pd(dppf)Cl₂·DCM" refers to [1,1'-bis(diphenylphosphino)ferrocene]dichloropalladium complex dichloromethane; "Pearlman's catalyst" refers to palladium hydroxide on carbon or Pd(OH)₂/C; "psi" refers to pounds per square inch; "PyBOP" refers to (benzotriazol-1-yl-oxytripyrrolidinophosphonium hexafluorophosphate); "PyBrOP" refers to bromo(tripyrrolidinyl)phosphoniumhexafluorophosphate; "rpm" refers to revolution per minute; "RT" refers to room temperature; "SCX" refers to strong cation exchange chromatography; "T3P" refers to 2,4,6-tri-*n*-propyl-1,3,5,2,4,6-trioxatriphosphorinane-2,4,6-trioxide or n-propylphosphonic anhydride; "TFA" refers to trifluoroacetic acid; "THF" refers to tetrahydrofuran; "Xantphos" refers to 4,5-bis(diphenylphosphino)-9,9-dimethylxanthene; "XantPhos-Pd-G2" refers to chloro[(4,5-bis(diphenylphosphino)-9,9-dimethylxanthene)-2-(2'-amino-1,1'-biphenyl)]palladium(II); "XPhos" refers to 2-dicyclohexylphosphino-2',4',6'-triisopropylbiphenyl; and "XPhos Pd G2" refers to chloro(2-dicyclohexylphosphino-2',4',6'-triisopropyl-1,1'-biphenyl)[2-(2'-amino-1,1'-biphenyl)]palladium(II);

The compounds of the present invention, or pharmaceutically acceptable salts thereof, may be prepared by a variety of procedures known to one of ordinary skill in the art, some of which are illustrated in the schemes, preparations, and examples below. The products of each step in the schemes below can be recovered by conventional methods well known in the art, including extraction, evaporation, precipitation, chromatography, filtration, trituration, and crystallization. In the schemes below, all substituents unless otherwise indicated, are as previously defined. The reagents and starting materials are readily available to one of ordinary skill in the art. Without limiting the scope of the invention, the following schemes, preparations, and examples are provided to further illustrate the invention.

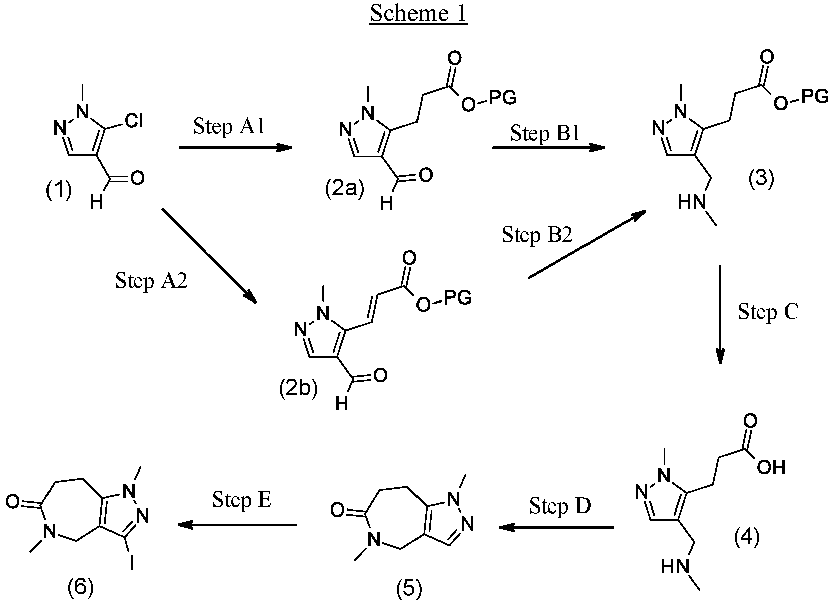

Scheme 1 illustrates the formation of a pyrazolo-[4,3,-c]azepin-6-one, compound (6). In Scheme1, Step A1, a substituted 5-chloro 4-carbaldehyde pyrazole (1) is coupled with a potassium-trifluoro boron propyl *t*-butoxy group under Suzuki palladium coupling conditions using a base such as cesium carbonate, in a solvent such as 1,4-dioxane and water, and a catalyst such as XPhos Pd G2 and heated to about 110 °C to give compound (2a). Palladium cross-coupling reactions are well known in the art and the skilled artisan will recognize that there are a variety of conditions useful for facilitating such cross-coupling reactions. Suitable palladium catalysts include XantPhos Pd G2, cataCXium^{®} A Pd G3, bis(triphenylphosphine)palladium(II) chloride, Pd(dppf)Cl₂, Pd₂dba₃, palladium tetrakistriphenylphosphine, and palladium(II) acetate. Suitable ligands if needed could include tricyclohexylphosphine and triphenyl phosphine. Suitable bases include potassium fluoride, cesium carbonate, sodium carbonate, potassium carbonate, sodium *t-*butoxide, and potassium phosphate tribasic monohydrate.

In Scheme 1, step B1, compound (2a) can undergo a reductive amination to provide the methylamino compound (3). The reductive amination can be accomplished using conditions well known in the art, such as adding methylamine to a solution of compound (2a) in DCM or a MeOH solution with a catalytic amount of an acid such as acetic acid and stirring for a suitable time such as about 1 hour. A suitable reducing agent is then added, such as sodium triacetoxyborohydride. Other reducing agents known in the art can be utilized such as NaBH₄ or LiBH₄ to provide the methylamino compound (3).

In Scheme 1, step C, the methylamino compound (3) can be deprotected under acidic conditions well known in the art such as using TFA or hydrochloric acid at RT to provide the carboxylic acid (4). "PG" is a protecting group developed for carboxylic acids. Such protecting groups are well known and appreciated in the art. The carboxylic acid (4) can then be cyclized with an amide coupling reagent to give compound (5). The internal amide cyclization can be accomplished in a polar aprotic solvent such as DMF at RT with a base such as DIPEA and a coupling reagent such as T3P. One skilled in the art will recognize that there are a number of methods and reagents for amide formation resulting from the reaction of carboxylic acids and amines. For example, the reaction of the amine with an appropriate carboxylic acid in the presence of a coupling reagent with or without an organic base such as DIPEA or triethylamine can provide compound (5). Coupling reagents include carbodiimides, such as DCC, DIC, EDCI or a carbonyldiimidazole such as CDI. Amide coupling additives, such as HOBt and HOAt can also be used to enhance the reaction. Additionally, uronium or phosphonium salts of non-nucleophilic anions, such as HBTU, HATU, PyBOP, and PyBrOP could be used in place of the more traditional coupling reagents. An additive such as DMAP may be used to enhance the reaction. Alternatively, a crude solution of compound 3 in EtOH can be treated with 2,2,2-trifluoroethanol in a continuous reaction system to deprotect and cyclize in situ and upon concentration gives compound (5).

Compound (5) can then be iodinated on the pyrazole using iodine and silver sulfate in a solvent such as EtOH to provide compound (6). Those skilled in the art will recognize that other halogens such as bromine and chlorine may be substituted in place of the iodine to provide a suitable corresponding halogenated compound (6), i.e., the iodine can also be bromine or chlorine, providing a suitable intermediate for use in Scheme 2 below.

Following alternative reaction steps in Scheme 1, in step A2, compound (1) can be treated with *tert*-butyl acrylate under Heck reaction conditions to form the substituted alkene of compound (2b) using a base such as *N,N*-dicyclohexylmethylamine in a solvent such as 1,4-dioxane and a suitable palladium catalyst, such as chloro[(tri-*tert-*butylphosphine)-2-(2-aminobiphenyl)] palladium(II)]. Typical catalysts used in Heck reactions are tetrakis(triphenylphosphine)palladium(0), palladium chloride, and palladium(II) acetate. Typical ligands are triphenylphosphine, tri-*tert*-butylphosphine, Xantphos, XPhos and bis(di-*tert*-butyl-phosphino)ferrocene.

In Scheme 1, step B2, compound (2b) can then be converted to compound (3) in an overall 2 step reaction. Methylamine can be added to compound (2b) in a solvent such as EtOH and stirred for about 90 minutes to effect formation of the imine. The alkene and imine can then be reduced under hydrogenation conditions using a suitable catalyst, such as Pearlman's catalyst, and hydrogenated to provide compound (3).

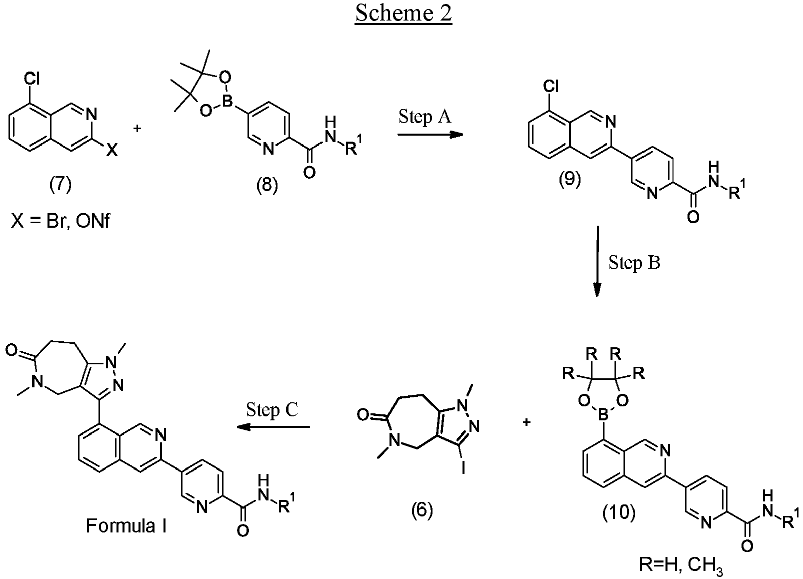

In Scheme 2, step A, compound (9) can be prepared from the boronic ester coupling of compound (8) with 3-bromo-8-chloro-isoquinoline, compound (7) (X=Br, ONf), under Suzuki cross coupling conditions analogous to the conditions as described for compound 2a in Scheme 1, step A1. For example, compound (7) is combined with compound (8) and treated with a base such as sodium carbonate or potassium carbonate in solvents such as 1,4-dioxane and water under nitrogen conditions using a catalyst such as Pd(dppf)Cl₂·DCM and heated to about 80-95 °C to provide compound (9). Alternatively, compound 8 can be generated in situ from 5-bromo-N-methylpyridine-2-carboxamide, bis(pinacolato)diboron, and KOAc in CPME using Pd(dppf)Cl₂ at 60-95 °C. After an appropriate reaction time of about 4-8 hours, the mixture is cooled and compound (9) is prepared as described above maintaining the solvent as CPME and using Pd(dppf)Cl₂ as a catalyst. In Scheme 2, step B, the chloride of compound (9) can be replaced using bis(pinacolato)diboron (for R = CH₃) or tetrahydroxydiboron (for R = H) under Miyaura borylation conditions to give compound (10). For example, KOAc can be used as the base, XPhos can be used as ligand, and XPhos Pd G2 can be used as the catalyst in a solvent such as 1,4-dioxane for (R = CH₃) and ethylene glycol and MeOH (for R = H). The reaction mixture is then heated to about 40 °C (for R = H) and to about 90 °C for (R = CH₃) to provide compound (10). Other catalysts that could be used are palladium(II) acetate or Pd₂(dba)₃ and other solvents can be toluene or 2-methyl-2-butanol.

In Scheme 2, step C, compound (10) can then be coupled with compound (6) under standard palladium cross coupling conditions as described in Scheme1, Step A1.

For example, using a suitable solvent, such as 1,4-dioxane or EtOH, a suitable base, such as potassium phosphate and an appropriate catalyst, such as Pd(dppf)Cl₂·DCM or Pd₂dba₃ with tricyclohexylphosphine as a ligand if needed and heating to about 50-95 °C provides the compounds of Formula I.

The following preparations and examples further illustrate the invention.

### Preparation 1

### tert-Butyl 3-(4-formyl-2-methyl-pyrazol-3-yl)propanoate

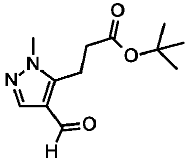

Scheme 1, step A1: A suspension of 5-chloro-1-methyl-1*H*-pyrazole-4-carbaldehyde (17.8 g, 119 mmol) and potassium;(3-*tert*-butoxy-3-oxo-propyl)-trifluoro-boranuide (38.0 g, 161 mmol) in 1,4-dioxane (298 mL) is treated with a solution of cesium carbonate (117 g, 358 mmol) in water (60 mL) and purged with nitrogen for 5 minutes. XPhos Pd G2 (9.4 g, 11.9 mmol) is added and the reaction mixture is purged again for 10 minutes and heated at 110 °C under nitrogen for 20 hours. The cooled reaction mixture is filtered through a pad of diatomaceous earth, washed with EtOAc (900 mL), DCM (200 mL) and concentrated in vacuo. The resulting oil is dissolved in EtOAc (1.2 L), washed with 1 M aq NaOH (3x200 mL) and brine (200 mL). The organic phase is dried over sodium sulphate, filtered, and evaporated to dryness. The crude material is purified by silica gel column chromatography eluting with a gradient of 0-50% hexanes to EtOAc to give the title compound (15.7 g, 53%) as a light-yellow oil. ES/MS (m/z): 239 (M+H), ¹H NMR (400.21 MHz, d₆-DMSO) δ 9.80 (s, 1H), 7.91 (s, 1H), 3.84 (s, 3H), 3.13 (t, J= 7.5 Hz, 2H), 2.58 (t, J= 7.5 Hz, 2H), 1.36 (s, 9H).

### Preparation 2

### tert-Butyl (E)-3-(4-formyl-2-methyl-pyrazol-3-yl)prop-2-enoate

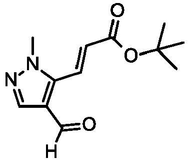

Scheme 1, step A2: 5-Chloro-1-methyl-1*H*-pyrazole-4-carbaldehyde (50.0 g, 329 mmol), *tert*-butyl acrylate (70 mL, 468 mmol) and *N,N-*dicyclohexylmethylamine (140 mL, 650 mmol) are dissolved in 1,4-dioxane (400 mL). The mixture is purged with nitrogen for 15 minutes, then chloro[(tri-*tert*-butylphosphine)-2-(2-aminobiphenyl)] palladium(II)] (3.46 g, 6.62 mmol) is added, purged again with nitrogen and the reaction mixture is stirred at 85 °C (internal temperature) for 16 hours then at 99 °C (internal temperature) for a further 3 hours. Heating is stopped, and when the internal temperature has fallen below 60 °C, the mixture is diluted with water (300 mL) and EtOAc (500 mL). The phases are separated, and the aq phase is diluted again with water (500 mL), after which a further organic phase forms. The phases are separated, and the aq extracted with EtOAc (250 mL). The combined organic phases are shaken with 10% aq citric acid (500 mL), filtered through diatomaceous earth, and washed with water (0.1 L) and EtOAc (250 mL). The organic layer is dried over MgSO₄, filtered, and concentrated under reduced pressure at 50 °C to give a thick red-orange oil (92 g). Heptane (500 mL) is added to the mixture while at 50 °C, then stirred overnight at RT during which time the initial oil converts to a solid. The resulting solid is collected by filtration and washed with 10% EtOAc/heptane (50 mL) followed by heptane (100 mL). The solid is dried in a vacuum oven (40 °C, 10 mbar) for 22 hours to give the title compound (57.1 g, 73%) as a cream solid. ES/MS (m/z): 237 (M+H), ¹H NMR (400.21 MHz, CDCl₃) δ 9.96 (s, 1H), 7.99 (s, 1H), 7.64 (d, J= 16.1 Hz, 1H), 7.02 (d, J= 16.1 Hz, 1H), 4.02 (s, 3H), 1.57 (s, 9H).

### Preparation 3

### tert-Butyl 3-[2-methyl-4-(methylaminomethyl)pyrazol-3-yl]propanoate

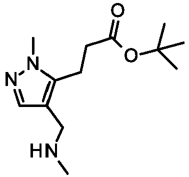

Scheme 1, step B1: Methanamine 2 M in THF (10.2 mL, 20.4 mmol) is added to a solution of *tert*-butyl 3-(4-formyl-2-methyl-pyrazol-3-yl)propanoate (0.81 g, 3.4 mmol) and acetic acid (0.2 mL, 3.4 mmol) in DCM (17 mL). The mixture is stirred at RT for 1 hour. Sodium triacetoxyborohydride (2.88 g, 13.6 mmol) is added and the reaction mixture is stirred at RT for 17 hours. The reaction mixture is diluted with saturated aq NaHCO₃ (30 mL) and DCM (30 mL). The aq phase is extracted with DCM (4x100 mL). The combined organic extracts are dried over sodium sulphate, filtered, and evaporated to dryness to give a brown oil. The crude material is purified by silica gel column chromatography eluting with a gradient of 0-70% DCM to DCM/7 N NH₃ in MeOH (9:1) to give the title compound (0.64 g, 73%) as an oil. ES/MS (m/z): 254 (M+H), ¹H NMR (400.21 MHz, d₆-DMSO) δ 7.22 (s, 1H), 3.72 (s, 3H), 3.42 (s, 2H), 2.85 (t, J= 7.7 Hz, 2H), 2.47 (t, J= 7.7 Hz, 2H), 2.24 (s, 3H), 1.38 (s, 9H).

### Alternate Preparation 3

### tert-Butyl 3-[2-methyl-4-(methylaminomethyl)pyrazol-3-yl]propanoate

Scheme 1, step B2: Methylamine (33% in EtOH, 85 mL, 683 mmol) is added to a solution of *tert*-butyl (E)-3-(4-formyl-2-methyl-pyrazol-3-yl)prop-2-enoate (57.1 g, 239 mmol) in EtOH (180 mL) and stirred at RT for 90 minutes. A slurry of Pearlman's catalyst, wet (20 wt% dry basis, 2.5 g, 18 mmol) in EtOH (60 mL) is charged to a 316 SS autoclave vessel under a nitrogen purge. The solution of imine prepared initially is added and rinsed with EtOH (60 mL). The autoclave is mounted and the vessel closed, purged and filled to 110 psi (7.1 bar) with hydrogen, then stirred at RT for 20 hours (no further hydrogen consumption after approx. 14 hours). The mixture is filtered through a pad of diatomaceous earth and rinsed with EtOH. The filtrate is concentrated under reduced pressure to a volume of approximately 200 mL. ES/MS and ¹H NMR of the solution are consistent with the expected compound. The EtOH solution is used without isolation and further purification of the title compound, assuming quantitative yield (65.9 g, 239 mmol)

Preparation 4

### 3-[2-Methyl-4-(methylaminomethyl)pyrazol-3-yl]propanoic acid

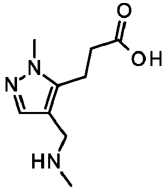

Scheme 1, step C: To a solution of *tert*-butyl 3-[2-methyl-4-(methylaminomethyl)pyrazol-3-yl]propanoate (9.6 g, 37.5 mmol) in DCM (150 mL) is added TFA (43 mL, 562 mmol) and the mixture is stirred at RT for 2 hours. The solution is concentrated under reduced pressure and traces of TFA are co-evaporated with ACN to give a colorless oil that is used in the next step without further purification, assuming quantitative yield (7.54 g, 37.5 mmol). ES/MS (m/z): 198 (M+H).

### Preparation 5

### 1,5-Dimethyl-7,8-dihydro-4H-pyrazolo[4,3-c]azepin-6-one

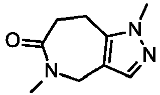

Scheme 1, step D: 3-[2-Methyl-4-(methylaminomethyl)pyrazol-3-yl]propanoic acid (7.54 g, 37.5 mmol) is dissolved in DMF (375 mL) and treated with DIPEA (24.2 g, 187 mmol) and T3P (50% in DMF) (43.8 mL, 75.0 mmol) dropwise and stirred at RT for 5 hours. The reaction mixture is diluted with water (800 mL) and the pH adjusted to pH 8 with sat. aq NaHCO₃. The solution is extracted with DCM/iPrOH (8:2) (8x250 mL). The combined organic extracts are dried over sodium sulphate, filtered, and evaporated to dryness. The residue is purified with SCX chromatography using the following conditions: The column is equilibrated with 2 volumes MeOH, the compound is dissolved in MeOH (5+5 mL), added to the column, washed with MeOH (6 volumes) and eluted with 7 N NH₃ in MeOH (250 mL). The basic fraction is evaporated to give the title compound (5.56 g, 83%) as an off-white solid. ES/MS (m/z): 180 (M+H). ¹H NMR (400.21 MHz, d₆-DMSO) δ 7.23 (s, 1H), 4.34 (s, 2H), 3.65 (s, 3H), 2.86 (s, 3H), 2.85-2.77 (m, 4H).

### Alternate Preparation 5

### 1,5-Dimethyl-7,8-dihydro-4H-pyrazolo[4,3-c]azepin-6-one

Scheme 1, step D: A solution of *tert*-butyl 3-[2-methyl-4-(methylaminomethyl)pyrazol-3-yl]propanoate in EtOH from Alternate Preparation 3 (approx. 239 mmol) is filtered through a 0.45 µm polypropylene membrane. The volume is adjusted to 0.3 L using EtOH and 2,2,2-trifluoroethanol (750 mL) is added to afford the Feed Solution. A 60 mL 316-SS tubular reactor (1/8" OD) with 4000 kPa backpressure regulator is flushed with a 3:1 mixture of 2,2,2-trifluoroethanol and EtOH (Flush Solvent). The reactor is heated to 220 °C and the Feed Solution is pumped through the reactor at a rate of 3 mL/min (residence time 20 minutes) using a chemically-resistant HPLC pump. After all the solution has been pumped into the reactor, the reactor is flushed with a 3:1 mixture of 2,2,2-trifluoethanol and EtOH (approximately 100 mL) at the same flowrate. The output solution and flush are concentrated under reduced pressure at 50 °C to give the crude title compound (48.38 g, 89% LCMS purity, approximately 20 wt% solvent by ¹H NMR, 186.3 mmol). ES/MS (m/z): 180 (M+H).

### Preparation 6

### 3-Iodo-1,5-dimethyl-7,8-dihydro-4H-pyrazolo[4,3-c]azepin-6-one

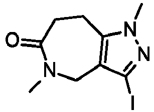

Scheme 1, step E: To a solution of 1,5-dimethyl-7,8-dihydro-4H-pyrazolo[*4,3-c*]azepin-6-one (5 g, 26.6 mmol) in EtOH (60 mL) is added iodine (pulverized, 10 g, 39.4 mmol) followed by silver sulfate (15.2 g, 48.7 mmol). The reaction mixture is stirred at RT for 48 hours. DCM (500 mL) is added and the suspension is stirred for 2 hours, the liquid is decanted and the solid is triturated with 20% iPrOH in DCM (600 mL) and stirred for 4 hours. The mixture is filtered, the solid is discarded and the filtrate is concentrated *in vacuo.* Aq NH₃ (32%, 150 ml) is added and the solution is extracted with DCM (2x350 mL). The combined organic extracts are washed with sat. Na₂S₂O₃, dried over sodium sulfate, filtered and concentrated *in vacuo* to give a solid. The solid is triturated in a 1:1 mixture of hexanes:MTBE (90 mL) for 2 hours. The solids are filtered and dried in an oven under vacuum for 16 hours at 45 °C to give the tittle compound (7.36 g, 89%) as a white solid. ES/MS (m/z): 306 (M+H), ¹H NMR (400.21 MHz, d₆-DMSO) δ 4.20 (s, 2H), 3.68 (s, 3H), 2.87 (s, 3H), 2.86-2.80 (m, 4H).

### Alternate Preparation 6

### 3-Iodo-1,5-dimethyl-7,8-dihydro-4H-pyrazolo[4,3-c]azepin-6-one

Scheme 1, step E: A solution of 1,5-dimethyl-7,8-dihydropyrazolo[*4,3-c*]azepin-6-one (31.6 g, 94% LCMS purity, 166 mmol) in EtOH (250 mL) is added to silver sulfate (62.3 g, 200 mmol). The solution is stirred under nitrogen. A solution of iodine monochloride in DCM (1.0 M, 220 ml, 220 mmol) is added in four approximately equal portions at 15 min intervals. An external water bath is used to reduce the exotherm. After stirring for 23 hours, a further portion of iodine monochloride in DCM (1.0 M, 33 ml, 33 mmol) is added. After stirring for a further 73.5 hours, the mixture is filtered through diatomaceous earth, and the filter cake is washed with DCM (300 mL). The filtrate and wash are stirred with a 1:1 mixture of 20% aq NaHSO₃ and sat. aq NaCl (400 mL) for 1.5 hours, then filtered through diatomaceous earth and the filter cake is washed with DCM (200 mL). The phases of the filtrate are separated, and the aq extracted with DCM (200 mL). The combined organic extracts are washed with sat. aq NaCl, dried over MgSO₄, and concentrated under reduced pressure at 50 °C to give a soft yellow residue (57 g). The residue is dissolved in DCM (250 mL), poured onto a DCM-washed silica pad (9 cm diameter x 5 cm depth) and eluted under suction using portions of 1:4 EtOH:DCM (500 mL). Fractions containing the target are concentrated under reduced pressure at 50 °C, and the resulting residue is triturated in MTBE (250 mL) at ambient temperature for approximately 16 hours. The solid is collected by filtration, washed with MTBE (0.1 L) and dried at 50 °C and 10 mbar for 8.5 hours to give the title compound as a finely-divided white powder (41.0 g, 80%). ES/MS (m/z): 306 (M+H).

### Preparation 7

### (8-Chloro-3-isoquinolyl) 1,1,2,2,3,3,4,4,4-nonafluorobutane-1-sulfonate

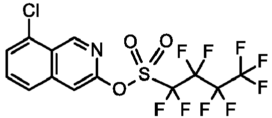

Potassium carbonate (75 g, 542.7 mmol) and 1,1,2,2,3,3,4,4,4-nonafluorobutane-1-sulfonyl fluoride (70 mL, 398.8 mmol) are added to 8-chloro-2*H*-isoquinolin-3-one (63 g, 350.7 mmol) in ACN (500 mL) and the resulting mixture is stirred at 60 °C for 15 hours. The mixture is filtered, and the solid is washed with further ACN. The filtrate is concentrated under reduced pressure. The residue is suspended in water (300 mL) and the pH is adjusted to >7 with sat. aq NaHCO₃ (20 mL). The suspension is stirred for 2 hours then filtered. The filter cake is washed with water and heptane, then dried under reduced pressure at 35 °C. The dried solid is taken up in DCM, silica gel (100 mL) is added, and the mixture is concentrated under reduced pressure. The resulting solid is loaded onto a silica pad (400 mL silica, previously washed with 1:4 EtOAc:n-hexanes) and eluted under suction using 1:4 EtOAc:n-hexanes (1.5 L). The eluate is concentrated under reduced pressure to approximately 1/20^{th} of the original volume, and n-hexanes (50 mL) is added to precipitate a solid. The solid is collected by filtration, and further crops of solid obtained by similar treatment of the filtrate (total 4 crops). The crops of solid are combined and dried under reduced pressure at 50 °C overnight to give the title compound as a white solid (50.82 g, 55.9%). ES/MS (m/z) (³⁵Cl/³⁷Cl): 462/464 (M+H); ¹H NMR (400.21 MHz, CDCl₃) δ 9.53 (s, 1H), 7.89-7.82 (m, 1H), 7.77-7.69 (m, 2 H), 7.62 (s, 1H); ¹⁹F{¹H} NMR (376.5 MHz, CDCl₃) δ -80.6 (m), -108.7 (m), -120.8 (m), -125.7 (m).

### Preparation 8

### 5-(8-Chloro-3-isoquinolyl)-N-methyl-pyridine-2-carboxamide

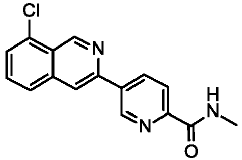

Scheme 2, step A: 3-Bromo-8-chloro-isoquinoline (500 mg, 2.06 mmol), *N-*methyl-5-(4,4,5,5-tetramethyl-1,3,2-dioxaborolan-2-yl)pyridine-2-carboxamide (584 mg, 2.23 mmol) and sodium carbonate (655 mg, 6.19 mmol) are suspended in 1,4-dioxane (8.2 mL) and water (1.6 mL). The mixture is purged with nitrogen (5 minutes). Pd(dppf)Cl₂·DCM (135 mg, 0.165 mmol) is added and the reaction is stirred at 95 °C for 23 hours. The mixture is cooled to RT, filtered over diatomaceous earth, and rinsed with EtOAc (200 ml) and DCM (100 mL). The filtrate is concentrated under vacuum and the residue is purified by silica gel column chromatography eluting with a gradient of 0-40% DCM to DCM/MeOH (9:1) to give the title compound (610 mg, 98%) as a light-brown solid. ES/MS m/z (³⁵Cl/³⁷Cl): 298/300 (M+H), ¹H NMR (400.21 MHz, d₆-DMSO) δ 9.69 (s, 1H), 9.46 (dd, J= 0.7, 2.2 Hz, 1H), 8.87-8.85 (m, 1H), 8.78 (d, J= 2.4 Hz, 1H), 8.77-8.76 (m, 1H), 8.20 (d, J= 8.0 Hz, 1H), 8.09 (d, J= 7.7 Hz, 1H), 7.93-7.83 (m, 2H), 2.87 (d, J= 4.8 Hz, 3H).

### Alternate Preparation 8

### 5-(8-Chloro-3-isoquinolyl)-N-methyl-pyridine-2-carboxamide

Scheme 2, step A: 5-Bromo-N-methylpyridine-2-carboxamide (10.0 g, 46.0 mmol), bis(pinacolato)diboron (15.5 g, 59.8 mmol) and KOAc (11.5 g, 115 mmol) in CPME (120 mL) are added together and the mixture is purged with nitrogen for approximately 10 minutes. Pd(dppf)Cl₂ (1.0 g, 1.3 mmol) is added and the mixture is heated to 60 °C. The nitrogen is stirred under nitrogen for approximately 4 hours at 81 °C (internal temperature). The temperature is increased to 94 °C (internal temperature), and after 45 minutes additional bis(pinacolato)diboron (2.5 g, 9.6 mmol) is added. Heating is continued for 4 hours, then the mixture is cooled to ambient temperature. Nitrogen-purged 2 M aq potassium carbonate (70 ml, 140 mmol) is added, followed after 10 minutes by (8-chloro-3-isoquinolyl) 1,1,2,2,3,3,4,4,4-nonafluorobutane-1-sulfonate (22.28 g, 47.8 mmol). The mixture is purged with nitrogen for approximately 10 minutes, then heated to an internal temperature of 84 °C. After 5 hours, Pd(dppf)Cl₂ (0.5 g, 0.7 mmol) is added, and heating is continued for 3 hours. Heating is stopped and the mixture is left to cool to 50 °C, then diluted with 2 M aq potassium carbonate (75 mL, 150 mmol) and EtOAc (120 mL). The mixture is cooled to ambient temperature and filtered. The filter cake is pressed down and washed with water (200 mL) and EtOAc (100 mL). The phases of the filtrate are separated, and the organic phase washed with 50 mL portions of 2 M aq potassium phosphate and sat. aq NaCl, dried over MgSO₄, filtered, and concentrated under reduced pressure. The residue is combined in DCM (300 mL) with the solid collected by filtration, and stirred vigorously for approximately 2 hours. The mixture is filtered, and the cake is washed with DCM (100 mL) and then discarded. A small aq phase is separated from the filtrate and discarded, and the organic phase is washed with brine then poured onto a silica pad (9 cm diameter x 2.5 cm depth) and eluted under suction with DCM (3x500 mL) and 1:9 acetone:DCM (4x500 mL). Fractions containing the target are combined and concentrated under reduced pressure, and the resulting residue is triturated in MTBE (250 mL) at 50 °C for 1 hour, then chilled in an ice-water bath for 1 hour. The solid is collected by filtration, washed with cold MTBE (2x50 mL) and dried under reduced pressure (45 °C, 10 mbar) for 18 hours to give the title compound as an off-white solid (7.51 g, 54.8%). ES/MS m/z (³⁵Cl/³⁷Cl): 298/300 (M+H),

### Preparation 9

### 5-(8-Chloro-3-isoquinolyl)-N-ethyl-pyridine-2-carboxamide

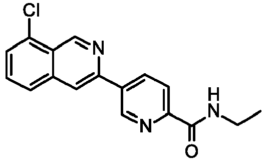

Scheme 2, step A: (8-Chloro-3-isoquinolyl) 1,1,2,2,3,3,4,4,4-nonafluorobutane-1-sulfonate_(500 mg, 1.08 mmol), N-ethyl-5-(4,4,5,5-tetramethyl-1,3,2-dioxaborolan-2-yl)pyridine-2-carboxamide (390 mg, 1.13 mmol) are suspended in 1,4-dioxane (11 mL) and 2 M aq potassium carbonate (1.62 mL, 3.24 mmol) is added. The solution is purged with nitrogen for 5 minutes. Pd(dppf)Cl₂·DCM (44 mg, 0.054 mmol) is added, the solution purged again with nitrogen and the reaction is stirred at 90 °C for 15 hours. The mixture is cooled to RT, quenched with water and extracted with EtOAc (3x100 mL). The combined organic extracts are washed with brine, dried over anhydrous sodium sulfate, filtered, and evaporated to dryness to give a brown oil. The residue is purified by silica gel column chromatography eluting with a gradient of 0% to 70% n-hexane to EtOAc to give the title compound (275 mg, 81%) as a light-brown solid. ES/MS m/z (³⁵Cl/³⁷Cl): 312/314 (M+H), ¹H NMR (400.21 MHz, d₆-DMSO) δ 9.70 (t, J= 0.8 Hz, 1H), 9.47 (dd, J= 0.8, 2.2 Hz, 1H), 8.91 (t, J= 5.9 Hz, 1H), 8.79-8.76 (m, 2H), 8.20 (dd, J= 0.8, 8.2 Hz, 1H), 8.11-8.09 (m, 1H), 7.90-7.83 (m, 2H), 3.34-3.41 (m, 2H), 1.17 (t, J= 7.2 Hz, 3H).

### Preparation 10

### N-Methyl-5-[8-(4,4,5,5-tetramethyl-1,3,2-dioxaborolan-2-yl)-3-isoquinolyl]pyridine-2-carboxamide

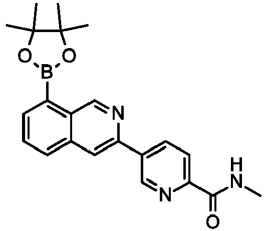

Scheme 2, step B: A mixture of 5-(8-chloro-3-isoquinolyl)-N-methyl-pyridine-2-carboxamide (750 mg, 2.27 mmol), bis(pinacolato)diboron (1.73 g, 6.80 mmol), KOAc (556 mg, 5.67 mmol) and XPhos (111 mg, 0.227 mmol) in 1,4-dioxane (32 mL) is purged with nitrogen. XPhos Pd G2 (182 mg, 0.227 mmol) is added and the mixture is purged with nitrogen. The mixture is stirred at 90 °C for 17 hours. The reaction mixture is cooled to RT, filtered through diatomaceous earth and washed with EtOAc (3x50ml). The mixture is concentrated, the residue is diluted with EtOAc (500 mL) and washed with water (2x80 mL). The organic layer is dried over sodium sulphate, filtered, and evaporated to give a brown oil that is purified by silica gel column chromatography eluting with a gradient of 0-70% hexanes to EtOAc to give the title compound (482 mg, 54 %) as a white solid. ES/MS (m/z): 390 (M+H), ¹H NMR (400.21 MHz, d₆-DMSO) δ 10.08 (s, 1H), 9.47 (dd, J= 0.7, 2.2 Hz, 1H), 8.87 (q, J= 4.7 Hz, 1H), 8.77 (dd, J= 2.2, 8.2 Hz, 1H), 8.68 (d, J= 0.6 Hz, 1H), 8.21-8.17 (m, 2H), 8.13 (dd, J= 1.2, 6.9 Hz, 1H), 7.86 (dd, J= 6.9, 8.2 Hz, 1H), 2.87 (d, J= 4.8 Hz, 3H), 1.43 (s, 12H).

### Preparation 11

### 5-[8-(1,3,2-Dioxaborolan-2-yl)-3-isoquinolyl]-N-methyl-pyridine-2-carboxamide

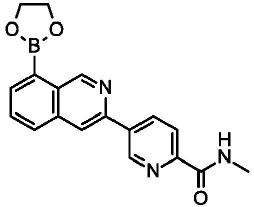

Scheme 2, step B: A mixture of 5-(8-chloro-3-isoquinolyl)-N methyl-pyridine-2-carboxamide (5.8 g, 19.3 mmol), tetrahydroxydiboron (2.9 g, 31 mmol), KOAc (5.0 g, 49.9 mmol) and XPhos Pd G2 (0.39 g, 0.49 mmol) in anhydrous ethylene glycol (15 ml) and MeOH (60 mL) is purged with nitrogen for 15 minutes. The mixture is then stirred at 40 °C for 14 hours. Water (150 mL) is added, and the mixture is stirred for 3 hours at ambient temperature. The solid is collected by filtration, washed with 1:3 MeOH:water (50 mL), water (50 mL) and MTBE (50 mL). The filter cake is dried in a vacuum oven (40 °C, 10 mbar) for 24 hours to give the title compound as a beige solid, consistent with the glycol ester as the major species (6.96 g, 91% LCMS purity, 99%). ES/MS (m/z): 308 (M+H for boronic acid); ¹H NMR (400.21 MHz, d₄-MeOH) δ 9.42 (s, 1H), 9.38 (d, J= 1.6 Hz, 1H), 8.66 (dd, J= 2.0, 8.4 Hz), 8.43 (s, 1H), 8.22 (dd, 1H, J= 0.8, 8.4 Hz, 1H), 8.09-8.03 (m, 1H), 7.87-7.78 (m, 1H), 3.62 (s, 3H), 3.03 (s, 3H).

### Preparation 12

### N-Ethyl-5-[8-(4,4,5,5-tetramethyl-1,3,2-dioxaborolan-2-yl)-3-isoquinolyl]pyridine-2-carboxamide

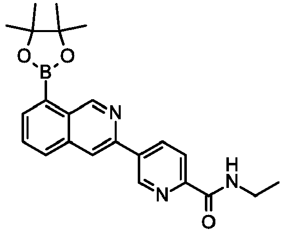

Scheme 2, step B: A mixture of 5-(8-chloro-3-isoquinolyl)-*N*-ethyl-pyridine-2-carboxamide (275 mg, 0.88 mmol), bis(pinacolato)diboron (672 mg, 2.65 mmol), KOAc (216 mg, 2.20 mmol) and XPhos (43 mg, 0.088 mmol) in 1,4-dioxane (11 mL) is purged with nitrogen. XPhos Pd G2 (69 mg, 0.088 mmol) is added and the mixture is purged with nitrogen and stirred at 90 °C for 21 hours. The mixture is cooled to RT, filtered through diatomaceous earth, and washed with EtOAc (200 ml). The mixture is concentrated, the residue is diluted with EtOAc (300 mL), and washed with water (2x80 mL). The organic layer is dried over sodium sulphate, filtered, and evaporated to give a brown oil that is purified by silica gel column chromatography eluting with a gradient of 0-50% hexanes to EtOAc to give the title compound (202 mg, 57 %) as an off-white solid. ES/MS (m/z): 404 (M+H), ¹H NMR (400.21 MHz, d₆-DMSO) δ 10.08 (s, 1H), 9.48 (dd, J= 0.7, 2.2 Hz, 1H), 8.90 (t, J= 6.0 Hz, 1H), 8.77 (dd, J= 2.2, 8.2 Hz, 1H), 8.67 (d, J= 0.5 Hz, 1H), 8.22-8.17 (m, 2H), 8.13 (dd, J= 1.2, 6.9 Hz, 1H), 7.86 (dd, J= 6.9, 8.2 Hz, 1H), 3.41-3.34 (m, 2H), 1.43 (s, 12H), 1.15-1.18 (m, 3H).

### Example 1

### 5-[8-(1,5-Dimethyl-6-oxo-7,8-dihydro-4H-pyrazolo[4,3-c]azepin-3-yl)-3-isoquinolyl]-N-methyl-pyridine-2-carboxamide

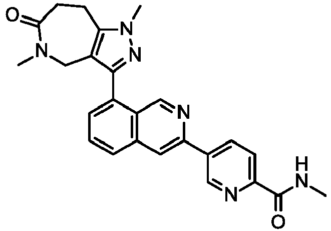

Scheme 2, step C: A solution of 3-iodo-1,5-dimethyl-7,8-dihydro-4H-pyrazolo[*4*,*3-*c]azepin-6-one (100 mg, 0.33 mmol) and *N*-Methyl-5-[8-(4,4,5,5-tetramethyl-1,3,2-dioxaborolan-2-yl)-3-isoquinolyl]pyridine-2-carboxamide (153 mg, 0.39 mmol) in 1,4-dioxane (2.2 mL) and *N*,*N-*dimethylacetamide (0.22 mL) is treated with 1 M aq potassium phosphate (0.98 mL 0.98 mmol) and purged with nitrogen for 5 minutes. Pd(dppf)Cl₂·DCM (27 mg, 0.033 mmol) is added and the reaction is stirred at 95 °C for 5.5 hours. The crude material is filtered through diatomaceous earth, washed with DCM and EtOAc and evaporated to dryness. The residue is purified with SCX chromatography using the following conditions: The column is equilibrated with 2 column volumes of MeOH, the compound is dissolved in MeOH (4 mL) and added to column, washed with MeOH (3 volumes) and eluted with 2 M NH₃ in MeOH (20 mL). Solvents are evaporated to give a brown oil. The oil is further purified by silica gel column chromatography, eluting with a gradient of 0-70% DCM to DCM/MeOH (9:1). The solid is dried in a vacuum oven at 40 °C for 16 hours to give the title compound (130 mg, 90%) as a light-brown solid. ES/MS (m/z): 441 (M+H), ¹H NMR (400.13 MHz, d₆-DMSO) δ 9.56 (s, 1H), 9.46 (d, J= 2.0 Hz, 1H), 8.85 (q, J= 4.9 Hz, 1H), 8.76 (dd, J= 2.0, 8.3 Hz, 1H), 8.73 (s, 1H), 8.19 (d, J= 8.3 Hz, 1H), 8.13 (d, J= 8.3 Hz, 1H), 7.93 (dd, J= 7.1, 8.3 Hz, 1H), 7.58 (dd, J= 1.2, 7.1 Hz, 1H), 4.33 (s, 2H), 3.85 (s, 3H), 3.03-2.91 (m, 4H), 2.87 (d, J= 4.9 Hz, 3H), 2.77 (s, 3H).

### Alternate Preparation Example 1

### 5-[8-(1,5-Dimethyl-6-oxo-7,8-dihydro-4H-pyrazolo[4,3-c]azepin-3-yl)-3-isoquinolyl]-N-methyl-pyridine-2-carboxamide

Scheme 2, step C: A mixture of 3-iodo-1,5-dimethyl-7,8-dihydro-4H-pyrazolo[*4,3-c*]azepin-6-one (9.3 g, 30.17 mmol) in 2-methyl-2-butanol (100 mL), *N-*methyl-5-[8-(4,4,5,5-tetramethyl-1,3,2-dioxaborolan-2-yl)-3-isoquinolyl]pyridine-2-carboxamide (approximately. 5 2 g, 68% LCMS purity, 10.5 mmol) and 2 M aq potassium phosphate (30 mL, 60 mmol) is purged with nitrogen for 30 minutes before addition of Pd(dppf)Cl₂ (0.71 g, 0.95 mmol). The mixture is heated in a heating block with 90 °C setpoint. The purge tube is removed when the block temperature reaches 60 °C. Further portions of *N*-methyl-5-[8-(4,4,5,5-tetramethyl-1,3,2-dioxaborolan-2-yl)-3-isoquinolyl]pyridine-2-carboxamide are added at 2 hours and 3.5 hours (total amount added: 15.45 g, 68% LCMS purity, 31.53 mmol). After a total of 20 hours heating, the mixture is cooled to ambient temperature and diluted with water (100 mL) and MTBE (250 mL). The mixture is filtered and the filtered solid washed with water (100 mL) and MTBE (100 mL). The phases of the filtrate are separated and heptane (400 mL) is added to the organic phase. The additional solid precipitate is collected by filtration after 1 hour and combined with the previously-collected solid. The combined solids are taken up in a mixture of DCM (500 mL), acetone (200 mL), and MeOH (100 mL) and concentrated onto diatomaceous earth under reduced pressure at 50 °C. The solid is loaded into a suitable cartridge and purified using silica gel column chromatography (750 g silica gel), eluting with a gradient of 0-15% EtOH/DCM. Target-containing fractions are combined and concentrated under reduced pressure to approximately 60 mL of a clear brown solution. The solution is stirred at 60 °C and seeded with a previously-prepared sample of the target. The resulting slurry is stirred further for 1 hour at 60 °C under a positive pressure of nitrogen, then cooled to ambient temperature. After 16 hours, the solid is collected by filtration and washed with ice-cold EtOH (35 mL). Additional material is isolated by concentration and further chromatography of the filtrate. The two crops of solid are combined and taken up in THF (200 mL) and stirred with dimercaptotriazine-functionalized metal scavenger resin (SiliCycle Inc, SiliaMetS^{®} DMT, 2 g, 1.2 mmol as dimercaptotriazine) at 40 °C for 67 hours. The scavenger is removed by filtration through a 0.45 µm polypropylene membrane filter, and the solid rinsed with THF (100 mL). The filtrate and wash are concentrated under reduced pressure at 50 °C, and solvent-swapped to EtOH (final volume approx. 50 mL), during which time a white slurry forms. The slurry is aged for 1 hour at 60 °C under a positive pressure of nitrogen, then cooled to ambient temperature. After 6 hours, the solid is collected by filtration and washed with ice-cold EtOH (25 mL). The solid is dried under reduced pressure for 30 hours (5 hours at 60 °C, 25 hours at 50 °C) to give the title compound as a white powder (8.95 g, 66%). ES/MS (m/z): 441 (M+H).

### Example 2

### 5-[8-(1,5-Dimethyl-6-oxo-7,8-dihydro-4H-pyrazolo[4,3-c]azepin-3-yl)-3-isoquinolyl]-N-ethyl-pyridine-2-carboxamide

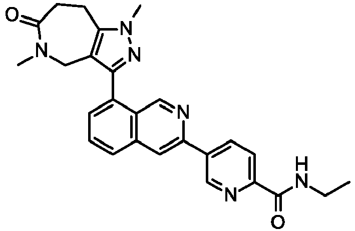

Scheme 2, step C: A solution of 3-iodo-1,5-dimethyl-7,8-dihydro-4H-pyrazolo[*4,3-c*]azepin-6-one (130 mg, 0.43 mmol), *N*-ethyl-5-[8-(4,4,5,5-tetramethyl-1,3,2-dioxaborolan-2-yl)-3-isoquinolyl]pyridine-2-carboxamide (353 mg, 0.49 mmol) in 1,4-dioxane (2.85 mL) and *N,N*-dimethylacetamide (0.28 mL) is treated with 1 M aq potassium phosphate (1.27 mL, 1.27 mmol) and purged with nitrogen for 5 minutes. Pd(dppf)Cl₂·DCM (35 mg, 0.042859 mmol) is added and the reaction is stirred at 90 °C for 17 hours. The material is cooled to RT, filtered through diatomaceous earth, washed with DCM and EtOAc, and evaporated to dryness. The residue is purified with SCX chromatography using the following conditions: The column is equilibrated with 2 column volumes of MeOH, the compound is dissolved in MeOH (4 mL) and added to the column, washed with MeOH (3 volumes) and eluted with 2 M NH₃ in MeOH (20 mL). The solvents are evaporated to give a brown oil. The material is further purified by reverse phase chromatography using a 10 mM ammonium bicarbonate buffer, pH=9 and eluting with a gradient of 20% to 60% ACN in buffer to give the title compound (83 mg, 42%) as a light-brown solid. ES/MS (m/z): 455 (M+H), ¹H NMR (400.13 MHz, d₆-DMSO) δ 9.57 (s, 1H), 9.46 (dd, J= 0.7, 2.2 Hz, 1H), 8.89 (t, J= 6.1 Hz, 1H), 8.76 (dd, J= 2.2, 8.3 Hz, 1H), 8.73 (s, 1H), 8.20-8.18 (m, 1H), 8.13 (d, J= 8.6 Hz, 1H), 7.93 (dd, J= 7.1, 8.3 Hz, 1H), 7.58 (dd, J= 1.1, 7.1 Hz, 1H), 4.33 (s, 2H), 3.85 (s, 3H), 3.41-3.34 (m, 2H), 3.04-2.91 (m, 4H), 2.77 (s, 3H), 1.17 (t, J= 7.2 Hz, 3H).

### Alternate Preparation Example 2

### 5-[8-(1,5-Dimethyl-6-oxo-7,8-dihydro-4H-pyrazolo[4,3-c]azepin-3-yl)-3-isoquinolyl]-N-ethyl-pyridine-2-carboxamide

Scheme 2, step C: A mixture of 3-iodo-1,5-dimethyl-7,8-dihydro-4H-pyrazolo[*4,3-c*]azepin-6-one (3.50 g, 11.36 mmol) and *N*-ethyl-5-[8-(4,4,5,5-tetramethyl-1,3,2-dioxaborolan-2-yl)-3-isoquinolyl]pyridine-2-carboxamide (4.71 g, 11.56 mmol) in EtOH (35 mL) and 2 M aq. potassium phosphate (11 mL, 22 mmol) is purged with nitrogen for 15 minutes. Tricyclohexylphosphine (0.19 g, 0.68 mmol) and Pd₂dba₃ (0.21 g, 0.22 mmol) are added, and the nitrogen purge is continued for a further 10 minutes. Heating is started, and the purge tube is removed once the heating block has reached 50 °C. The mixture is refluxed for 4 hours, then cooled to RT. Dimercaptotriazine-functionalized metal scavenger resin (SiliCycle, Inc, SiliaMetS^{®} DMT, loading 0.60 mmol/g, 2.0 g, 1.2 mmol) is added, and the mixture stirred at 50 °C for 3.5 hours, then cooled to RT. Water (120 mL) is added, and the mixture stirred at RT for 30 minutes. The mixture is filtered through diatomaceous earth, and the pad rinsed with 1:3 EtOH/water (40 mL). The filtrate is diluted with water (400 mL) and stirred at ambient temperature for 1.5 hours. The resulting white solid is collected by filtration, washed with 1:6 EtOH/water (70 mL), water (100 mL), and dried under reduced pressure for 16 hours at 50 °C to give the crude title compound. The crude solid is taken up in EtOH (40 mL) and DCM (10 mL) and filtered through diatomaceous earth. The filtrate is concentrated under reduced pressure at 50 °C until approximately 20 mL of solution remains. The solution is stirred in a 70 °C heating block, and heptane (20 mL) is added over approximately 2 minutes. The mixture is stirred in the heating block for 1 hour, during which time a white solid is formed. Heating is stopped, and the mixture is left to cool with stirring. After 6 hours, the solid is collected by filtration and washed with 1: 1 EtOH/heptane (10 mL) and heptane (10 mL) and then dried under reduced pressure at 50 °C for 13 hours to give the title compound (4.2 g, 81%) as a white solid. ES/MS (m/z): 455 (M+H).

### Biological Assays

### NanoBRET Assay

HEK293 cells are seeded at 10 × 10⁶ cells/16 mL in DMEM + 10% FBS in a T-75 flask and left to attach for 4-6 hours. Transfection reagents are diluted in Opti-MEM media (no phenol red, no FBS) and 8% Fugene HD containing expression plasmids at a final concentration of the following: 1: Histone H3.3 HaloTag DNA (Promega, 0.02 µg) + NanoLuc-CREBBP-BD (Promega, 0.0002 µg); 2: Histone H3.3 HaloTag DNA (Promega, 0.02 µg) + NanoLuc-EP300-BD (Promega, 0.0002 µg); 3: Histone H4 HaloTag DNA (0.02 µg) + NanLuc-BRD4 Full Length (Promega, 0.0002 µg). The DNA/Fugene mixtures are left at RT for 10 minutes. Transfection mix (800 µL) is added per flask and immediately swirled to cover the cells. The plates are returned to the incubator for overnight transfection. The next day, HaloTag NanoBRET 618 ligand (20 µL/vial of 0.1 mM) is thawed at RT. Culture Media is removed from transfected cell flasks and are washed with PBS (5 mL) and treated with trypsin (3 mL) at RT to detach cells. Trypsinyzed cells are resuspended in DMEM + 10% FBS (7 mL) and collected in a conical tube (50 mL). The cells are spun at 1500 rpm for 5 minutes then resuspended in Opti-MEM (no phenol red) + 4% FBS. HaloTag NanoBRET 618 ligand (1 µL of 0.1 mM) is added to cells at 100 nm final concentration. Cells are added to white, opaque bottom 96-well plates (Costar 3917) and incubated overnight at 37 °C/5% CO₂.

Compound is serially diluted and added to 96-well plates (2 µL, 10 mM, 3-fold dilutions, 100% DMSO). Media (98 µL)/4% FBS is added to the compound plate for a final concentration of 200 µM, 2% DMSO. Control Compound Plates (MIN) are prepared at 200 µM, 2% DMSO (20 µM final, 0.2% final) by adding reference inhibitor (10 µL) to media (490 µL)/4% FBS. MIN reference inhibitor (50 µL) is added into control plate column 1A thru 1H. MAX Plates are prepared at 2% DMSO (0.2% final concentration) by adding DMSO (10 µL) to media (10 µL)/4% FBS. Maximun (50 µL) is added into control plate column 12A thru 12H. The cells are treated with 10 µL/well of serial diluted Example 1. The cell plates are incubated at 37 °C/5% CO₂. CREBBP/EP300 plates are read after 4 hours incubation and the BRD4 plates are read after overnight incubation. To develop plates for reading, allow all reagents to reach RT before use. 5 mM Stock of NanoBRET Nano-Glo Substrate is diluted to 50 µM in Opti-MEM (without phenol red, no serum). Nano-Glo substrate (25 µL/well of 50 µM) is added. The plate is shaken for 1 minute to mix and within 10 minutes, read on Envision plate reader with the following settings: Mirror: Luminescence; Emission filter: E600LP; 2nd emission filter: D460/50; Measurement height (mm): 6.5; Measurement time (s): 0.2 second.

**Table 1**

| Compound | hBRD4 IC₅₀ (µM) | hCREBBP IC₅₀ (nM) | hEP300 IC₅₀ (nM) |
|---|---|---|---|
| Example 1 | 5.57 ± 4.10 | 16.0 ± 6.64 | 15.1 ± 2.75 |
| | n=6 | n=6 | n=6 |
| Example 2 | 11.8 | 18.6 ± 4.09, n=2 | 26.1 ± 16.5, n=2 |

| | | | |
|---|---|---|---|
| Data is shown as mean + Standard Deviation | | | |

As shown in Table 1, the data demonstrates that the compounds of Example 1 and Example 2 potently inhibit CREBBP and EP300 *in vitro.* In addition, Table 1 shows that the compounds of Example 1 and Example 2 exhibit selectivity for CREBBP/EP300 over the BRD4 bromodomain.

### Cell Proliferation Assay in 22Rv1

The purpose of the following cell proliferation assay is to detect whether the compound of Example 2 and combinations thereof have an effect on cell proliferation.

22Rv1 (purchased from ATCC CRL-2505) cells are seeded at a density of 1,000 cells per well in RMPI 10% FBS (20 µL volume) into a clear bottom 384-well cell culture plate. The plate is incubated at 37 °C and 5% CO₂. The cells are dosed the following day with the compound of Example 2 and the compound of Example 2 in combination with the known anti-cancer therapeutics found in the table below. An Echo 555 acoustic dispenser is used to prepare serial dilutions (1:3) of the compound of Example 2, and combinations thereof, in concentrations ranging from 60 µM to 0.003 µM. The cells are then dosed by addition of 5 µL from the serial dilution plate to the cell plate, producing a final DMSO concentration of 0.2% with a final concentration dose ranging from 20 µM to 0.001 µM for the compound of Example 2 and combinations thereof.

Media containing 0.2% DMSO is used for the maximum point and 2 µM staurosporine in the growth media containing 0.2% DMSO is used for the minimum point. After dosing with the compound of Example 2 and combinations thereof, the cell plates are incubated at at 37 °C and 5% CO₂. Seven days after test compound addition, plates are removed from the incubator and cold EtOH (96%, 65 µL) is added to each well. After 30 minutes, the media is removed and RNase (20 µL, 50 µg/mL) (Sigma) and a 1:1000 propidium iodide dilution in PBS are added to each well. The plates are sealed and incubated for 1 hour at room temperature and preserved from light. Plates are then scanned with ACUMEN EXPLORER^{™} [Laser-scanning fluorescence microplate cytometer manufactured by TTP LABTECH LTD]. The number of 22Rv1 cells is evaluated through an estimated number of cells, calculated through the area parameter (ratio of total area of the total cells population), a designated range of peak intensity of FL-3 (PI), and the mean area of the single cell population defined by perimeter. Data is then processed through an NGR screening tool. Data is analyzed using a 4-parameter nonlinear logistic equation (four-parameter logistic concentration-response curve): Y = bot + [(top-bot)/1+(x/IC₅₀)slope] where Y = % inhibition, X = concentration yielding y% inhibition, "bot" = minimum value of y attained by curve, "top" = maximum value of y attained by curve, and "slope" = steepness of curve at IC₅₀. %Inh = [(median Max- x/ median Max - median Min)] · 100.

The % inhibition of the compound of Example 2 and combinations thereof, in 22Rv1 prostate cancer cells at various concentrations are illustrated in Tables 2 and 3 below.

**Table 2**

| | **Cell Proliferation % Inhibition (Concentration)** | | |
|---|---|---|---|
| **Combination Partner** | **Partner** | **Example 2** | **Partner + Example 2** |
| Darolutamide | 6.18 (2.2 µM) | 19.34 (2.2 µM) | 36.0 (1.1/1.1 µM) |
| Enzalutamide | 0.41 (3.3 µM) | 46.3 (3.3 µM) | 59.09 (3.3/3.3 µM) |
| Abemaciclib | 62.4 (37 nM) | 35.37 (370 nM) | 82.1 (37/370 nM) |
| Alpelisib | 45.34 (370 nM) | 18.68 (370 nM) | 69.72 (370/370 nM) |

**Table 3**

| | **Cell Proliferation % Inhibition (Concentration)** | | |
|---|---|---|---|
| **Combination Partner** | **Partner** | **Example 2** | **Partner + Example 2** |
| Darolutamide | 6.69 (740 nM) | 12.25 (740 nM) | 25.41 (370/370 nM) |
| Enzalutamide | -0.12 (370 nM) | 31.53 (370 nM) | 41.82 (370/370 nM) |
| Abemaciclib | 34.32 (12 nM) | 17.44 (120 nM) | 73.08 (12/120 nM) |
| Alpelisib | 33.07 (120 nM) | 9.62 (120 nM) | 75.54 (120/120 nM) |

The results of this assay demonstrate the anti-proliferative activity of the compound of Example 2 and the compound of Example 2 in combination with the anti-cancer therapeutics darolutamide, enzalutamide, abemaciclib, and alpelisib, in 22Rv1 (ARFL/ARV7; PIK3CAmut) prostate cancer cells. As seen in Table 2, cell proliferation is inhibited by the compound of Example 2. Additionally, cell proliferation is inhibited by the compound of Example 2 in combination with the anti-cancer therapeutics described in Table 2. Similarly, as seen in Table 3, cell proliferation is inhibited by the compound of Example 2. Additionally, cell proliferation is inhibited by the compound of Example 2 in combination with the anti-cancer therapeutics described in Table 3.

### Cell Growth Inhibition Assay

Tumor cell lines are plated onto 96-well, black wall poly-D-lysine plates (Corning # 354640), and incubated at 37 °C, 5% CO₂ for 18-24 hours. Compound was added at 1:3 dilutions in DMSO and cells were incubated at 37 °C, 5% CO₂ for 7 days. For adherent cell lines, cells were fixed and stained with propidium iodide and scanned on Acumen (channel 3 setting). For suspension cell lines, cell growth was monitored using Cell Titer-Glo reagent.

**Table 4**

| **Cell Line** | **Growth Inhibition (µM)** | |
|---|---|---|
| | **Example 1** | **Example 2** |
| SW780 | 0.001 | 0.004 |
| NCI-H1048 | 0.010 | 0.018 |
| NCI-H211 | 0.036 | 0.065 |
| EOL-1 | 0.0008 | 0.001 |
| MDA-MB-453 | 0.028 | 0.035 |
| Kasumi (AML1-ETO) | ND | 0.008 |
| SKNO-1 (AML1-ETO) | ND | 0.018 |
| KG-1a | 0.037 | 0.002 |
| THP-1 | ND | 0.029 |
| KG-1 | ND | 0.083 |
| MOLM-16 | ND | 0.129 |
| OCI-AML2 | ND | 0.207 |
| OCI-AML3 | ND | 0.226 |
| GDM1 | ND | 0.28 |
| HL-60 | ND | 0.328 |

Table 4 illustrates the growth inhibition of in cancer cell lines by the compounds of Example 1 and Example 2.

### BromoKdELECT^{®} Platform Assay

The purpose of this assay is to evaluate the *in vitro* target engagement and selectivity (https://www.discoverx.com/services/drug-discovery-development-services/epigenetic-profiling/bromoscan-epigenetic-profiling/bromokdelect). Assay measurement is based on the amount of bromodomain captured in test and control samples by using quantitative PCR technology that detects the DNA label associated to bromodomain. Compounds that bind the bromodomain active site prevents its binding to the immobilized ligand and will reduce the amount of bromodomain captured on the solid support. However, molecules that do not bind the bromodomain have no effect on the amount of protein captured on the solid support. Thus, compound activity is monitored by measuring the amount of bromodomain captured in test versus control samples by using qPCR. Binding affinity for test compound-bromodomain interactions are calculated by measuring the amount of bromodomain captured on the solid support as a function of the test compound concentration.

Binding affinity (Kd) of the compounds of Examples 1 and 2 towards CREBBP, EP300 and BRD4 (Bromodomain 1 (BD1) and Bromodomain 2 (BD2)) are determined in an 11 points dose-response study (See Table 5). Data is represented as a mean from a duplicate experiment.

**Table 5**

| **Gene** | **Example 1 K*_{d}* (nM)** | **Example 2 K*_{d}* (nM)** |
|---|---|---|
| EP300 | 0.16 | 0.095 |
| CREBBP | 1.1 | 0.325 |
| BRD4 (BD1) | 2000 | 2650 |
| BRD4 (BD1, BD2) | 4400 | 7400 |
| BRD4 (BD2) | 6700 | 6800 |

As shown in Table 5, the compound of Example 1 exhibits a binding affinity of 0.16 nM for EP300 and a binding affinity of 1.1 nM for CREBBP. In addition, as shown in Table 5, the binding affinity of the compound of Example 1 is selective for CREBBP/EP300 over the BRD4 bromodomain. Also shown in Table 5, the compound of Example 2 exhibits a binding affinity of 0.095 nM for EP300 and a binding affinity of 0.325 nM for CREBBP. In addition, as shown in Table 5, the binding affinity of the compound of Example 2 is selective for CREBBP/EP300 over the BRD4 bromodomain.

## Claims

1. A compound of the formula: 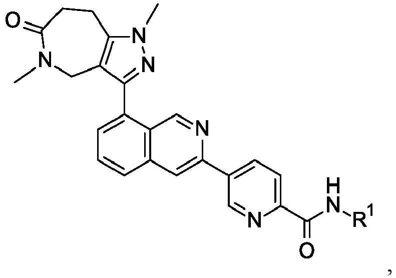
wherein R¹ is -CH₃ or -CH₂CH₃;
or a pharmaceutically acceptable salt thereof.

2. The compound according to claim 1 wherein R¹ is -CH₃; or a pharmaceutically acceptable salt thereof.

3. The compound according to claim 1 wherein R¹ is -CH₂CH₃; or a pharmaceutically acceptable salt thereof.

4. The compound according to claim 2 which is:

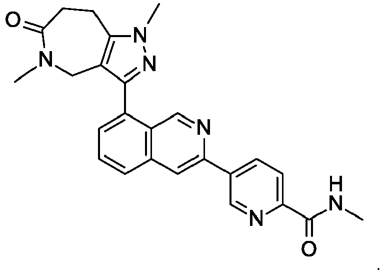

5. The compound according to claim 3 which is:

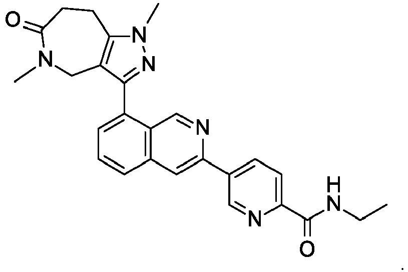

6. A compound or salt according to any one of claims 1-5, wherein the compound and/or salt are crystalline.

7. A compound, or a pharmaceutically acceptable salt thereof, according to any one of claims 1 to 6 for use in the treatment of cancer.

8. A compound , or a pharmaceutically acceptable salt thereof, for use according to claim 7, wherein the cancer is selected from squamous cell cancers, bladder urothelial cancer, breast cancer, colon cancer, diffuse large B cell lymphoma (DLBCL), follicular lymphoma, marginal zone lymphoma, upper tract urothelial cancer, endometrial carcinoma, uterine endometrioid carcinoma, SCLC, prostate cancer, lymphoma, esophageal cancer, lung cancer, bladder cancer, squamous histology, uterine endometrioid, HPV driven cancers, AML-ETO translocation related conditions, androgen receptor positive related conditions, myc overexpression related conditions, acute myeloid leukemia, acute lymphoblastic leukemia, multiple myelomas, human papillomavirus driven cancer, and prostate cancer.

9. A compound, or a pharmaceutically acceptable salt thereof, for use according to claim 8, wherein the squamous cell cancers are selected from anal cancer, bladder cancer, cervical cancer, head and neck cancer, lung cancer, and oropharynx cancer.

10. A pharmaceutical composition, comprising a compound or a pharmaceutically acceptable salt thereof according to any one of claims 1 to 6 with one or more pharmaceutically acceptable carriers, diluents, or excipients.

## Patentansprüche

1. Verbindung der Formel: 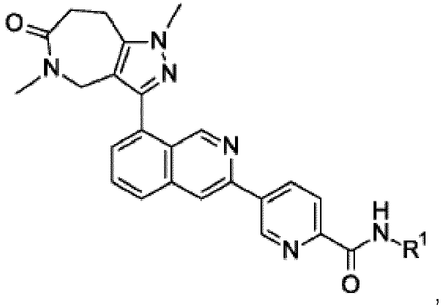
wobei R¹ -CHs oder -CH₂CH₃ ist;
oder ein pharmazeutisch unbedenkliches Salz davon.

2. Verbindung nach Anspruch 1, wobei R¹ -CHs ist; oder ein pharmazeutisch unbedenkliches Salz davon.

3. Verbindung nach Anspruch 1, wobei R¹ -CH₂CH₃ ist; oder ein pharmazeutisch unbedenkliches Salz davon.

4. Verbindung nach Anspruch 2, die: 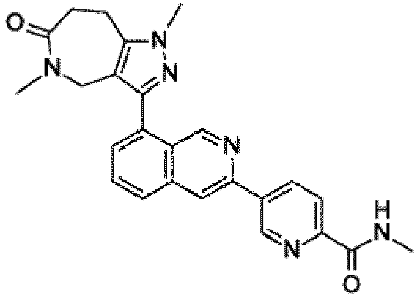 ist.

5. Verbindung nach Anspruch 3, die: 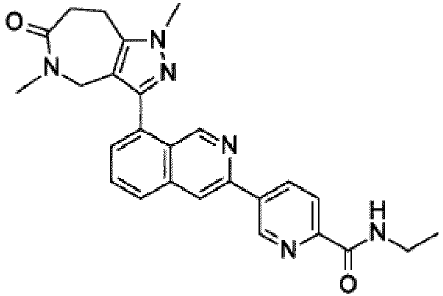 ist.

6. Verbindung oder Salz nach einem der Ansprüche 1-5, wobei die Verbindung und/oder das Salz kristallin sind.

7. Verbindung oder ein pharmazeutisch unbedenkliches Salz davon nach einem der Ansprüche 1 bis 6 zur Verwendung bei der Behandlung einer Krebserkrankung.

8. Verbindung oder ein pharmazeutisch unbedenkliches Salz davon zur Verwendung nach Anspruch 7, wobei die Krebserkrankung aus Plattenepithelkrebserkrankungen, Urothelkrebs der Blase, Brustkrebs, Dickdarmkrebs, diffusem großzelligem B-Zell-Lymphom (DLBCL), follikulärem Lymphom, Randzonenlymphom, Urothelkrebs des oberen Harntrakts, Endometriumkarzinom, Endometrioidkarzinom des Uterus, SCLC, Prostatakrebs, Lymphom, Speiseröhrenkrebs, Lungenkrebs, Blasenkrebs, Plattenepithelhistologie, Endometrioid des Uterus, von HPV verursachten Krebserkrankungen, mit einer AML-ETO-Translokation zusammenhängenden Leiden, mit einem positiven Androgenrezeptor zusammenhängenden Leiden, mit einer Myc-Überexpression zusammenhängenden Leiden, akuter myeloischer Leukämie, akuter lymphatischer Leukämie, multiplen Myelomen, von dem humanen Papillomavirus verursachter Krebserkrankung und Prostatakrebs ausgewählt ist.

9. Verbindung oder ein pharmazeutisch unbedenkliches Salz davon zur Verwendung nach Anspruch 8, wobei die Plattenepithelkrebserkrankungen aus Analkrebs, Blasenkrebs, Gebärmutterhalskrebs, Kopf- und Hals-Krebs, Lungenkrebs und Mund- und Rachenkrebs ausgewählt sind.

10. Pharmazeutische Zusammensetzung, umfassend eine Verbindung oder ein pharmazeutisch unbedenkliches Salz davon nach einem der Ansprüche 1 bis 6 mit einem oder mehreren pharmazeutisch unbedenklichen Trägerstoffen, Verdünnungsmitteln oder Hilfsstoffen.

## Revendications

1. Composé de formule : 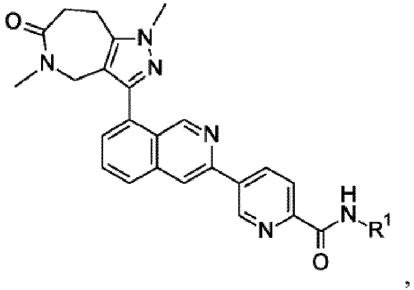
dans laquelle R¹ est -CHs ou -CH₂CH₃ ;
ou un sel pharmaceutiquement acceptable de celui-ci.

2. Composé selon la revendication 1, dans lequel R¹ est -CH₃ ; ou un sel pharmaceutiquement acceptable de celui-ci.

3. Composé selon la revendication 1, dans lequel R¹ est -CH₂CH₃ ; ou un sel pharmaceutiquement acceptable de celui-ci.

4. Composé selon la revendication 2 qui est :

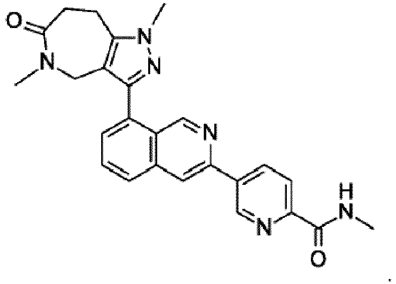

5. Composé selon la revendication 3 qui est :

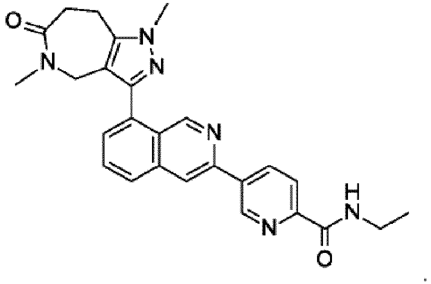

6. Composé ou sel selon l'une quelconque des revendications 1 à 5, dans lequel le composé et/ou le sel sont cristallins.

7. Composé, ou un sel pharmaceutiquement acceptable de celui-ci, selon l'une quelconque des revendications 1 à 6, pour une utilisation dans le traitement du cancer.

8. Composé, ou sel pharmaceutiquement acceptable de celui-ci, pour une utilisation selon la revendication 7, dans lequel le cancer est choisi parmi les cancers spino-cellulaires, le cancer urothélial de la vessie, le cancer du sein, le cancer du côlon, le lymphome B diffus à grandes cellules (DLBCL), le lymphome folliculaire, le lymphome de la zone marginale, le cancer urothélial des voies supérieures, le carcinome de l'endomètre, le carcinome endométrioïde utérin, le SCLC, le cancer de la prostate, le lymphome, le cancer de l'oesophage, le cancer du poumon, le cancer de la vessie, l'histologie épidermoïde, l'endométrioïde utérin, les cancers liés au VPH, les affections liées à la translocation AML-ETO, les affections positives aux récepteurs des androgènes, les affections liées à la surexpression de myc, la leucémie myéloïde aiguë, la leucémie lymphoblastique aiguë, les myélomes multiples, le cancer lié au papillomavirus humain et le cancer de la prostate.

9. Composé, ou sel pharmaceutiquement acceptable de celui-ci, pour une utilisation selon la revendication 8, dans lequel les cancers spino-cellulaires sont choisis parmi le cancer anal, le cancer de la vessie, le cancer du col de l'utérus, le cancer de la tête et du cou, le cancer du poumon et le cancer de l'oropharynx.

10. Composition pharmaceutique comprenant un composé ou un sel pharmaceutiquement acceptable de celui-ci selon l'une quelconque des revendications 1 à 6 avec un ou plusieurs supports, diluants ou excipients pharmaceutiquement acceptables.
